# DEMANDE DE BREVET EUROPEEN

(11) **EP 3 213 704 A1**
(43) Date de publication de la demande: **06.09.2017**
(21) Numéro de dépôt: 17158258.8
(22) Date de dépôt: 27.02.2017
(51) Int. Cl.: A61B 17/70, A61F 2/44, A61F 2/30

(54) **SYSTÈME D`IMPLANTS D`ARTHRODÈSE RACHIDIENNE**

(30) Priorité: 26.02.2016 FR 1651637
(71) Demandeur: LDR Medical, 10300 Sainte-Savine (FR)
(72) Inventeur: MERCIER, Alexis, 10390 VERRIERES (FR); LEQUETTE, Samuel, 33600 PESSAC (FR)
(74) Mandataire: Debay, Yves

(57) **Abrégé**

La présente invention concerne un système d'arthrodèse rachidienne comportant au moins deux types d'implants parmi les trois types suivants :
- Un implant intersomatique (IS), comportant au moins un passage (40) complémentaire d'au moins un dispositif d'ancrage(1) ;
- Un implant interépineux (IE), comportant au moins deux ailes aptes à longer une partie des épines (EI, ES) vertébrales ;
- Un implant facettaire (IF) comportant une fixation osseuse.

## Description

### DOMAINE TECHNIQUE DE L'INVENTION

La présente invention concerne le domaine des implants rachidiens, en particulier pour l'arthrodèse d'au moins deux structures vertébrales. La présente invention concerne plus particulièrement un système d'implants rachidiens destiné à assurer une fusion d'au moins deux vertèbres adjacentes (i.e., arthrodèse).

### ARRIERE-PLAN TECHNOLOGIQUE DE L'INVENTION

Un problème dans le domaine des implants concerne la croissance osseuse et notamment l'arthrodèse, c'est-à-dire la fusion osseuse de deux structures, comme par exemple des vertèbres. En effet, on cherche parfois à obtenir une fusion d'au moins deux vertèbres, par exemple lorsqu'au moins un de leurs disques intervertébraux adjacents est lésé. Il est connu de l'art antérieur diverses techniques d'arthrodèse, reposant sur divers types d'implants, comme par exemple les cages intersomatiques (ou d'arthrodèse) insérées à la place d'un disque pour promouvoir la croissance osseuse, ou les plaques d'arthrodèse fixées sur deux vertèbres pour les immobiliser et permettre l'arthrodèse, ou encore les barres d'ostéosynthèse ou d'arthrodèse, utilisées pour immobiliser les vertèbres, auxquelles elles sont généralement reliées par des vis pédiculaires ou des crochets, ou enfin les implants inter-épineux insérés entre les épines des vertèbres (ou « apophyses épineuses ») pour les immobiliser et faciliter ainsi la fusion. Les barres ou plaques sont généralement utilisées en complément de cages intersomatiques qui permettent une fusion étendue puisque située au niveau des corps vertébraux. Cette utilisation complémentaire découle d'un problème connu dans le domaine qui est de stabiliser le niveau vertébral à traiter. Il est également connu, notamment au niveau lombaire et sacré, des solutions utilisant des implants facettaires (interfacettaires ou transfacettaires) permettant de fixer les facettes articulaires dans le but d'obtenir une fusion.

Généralement, ces solutions visent à résoudre, en outre, le problème de la stabilité de l'implant et des structures vertébrales traitées. Il est nécessaire qu'un implant soit stable dans son site d'implantation, en particulier lorsqu'une arthrodèse est souhaitée puisque cette dernière doit avoir lieu dans une position relative des éléments du rachis qui soit optimale (telle que souhaitée par le chirurgien). Une stabilisation et/ou un verrouillage de l'implant est (sont) donc souvent préférable(s). Diverses solutions de l'art antérieur visent donc à fournir des implants d'arthrodèse stables.

Ainsi, il a été suggéré dans l'art antérieur qu'il serait souhaitable d'utiliser des implants complémentaires entre eux, comme par exemple des barres ou plaques d'ostéosynthèse en combinaison avec des cages intersomatiques. De plus, le fait que le rachis possède en fait trois grands axes de stabilité a été suggéré dans la littérature, notamment par F. Denis dans son article « The Three Column Spine and Its Significance in the Classification of Acute Thoracolumbar Spinal Injuries » dans le journal SPINE, 1983, vol. 8, n°8, pp 817-831, mais également par R. Louis dans son article « Spinal stability as defined by the three-column spine concept » dans le journal Anatomia Clinica, 1985, vol. 7, pp 33-42, puis par R. Roy-Camille dans son article « L'instabilité Rachidienne / Spinal Instability » dans le journal Rachis, 1994, vol.6, n° 2 pp 107-112 et plus récemment par Sabina MARCOVSCHI CHAMPAIN, dans sa thèse pour obtenir le grade de Docteur de l'École Nationale Supérieure d'Arts et Métiers, Spécialité "Biomécanique" à l'Ecole doctorale n° 432 (Sciences des Métiers de l'Ingénieur), document N° 2008-ENAM-0024.

Cependant, la plupart des solutions de l'art antérieur ne permettent pas de tirer avantage de ces enseignements de la littérature et de proposer un système d'arthrodèse rachidienne stable, notamment à cause des problèmes généraux de la facilité et/ou la rapidité de l'implantation et de l'invasivité des implants et des techniques chirurgicales qui en dépendent. En effet, on souhaite généralement que les implants puissent être implantés rapidement et/ou facilement, avec une invasivité minimale, c'est-à-dire que l'on cherche à limiter la taille des incisions et des dommages sur les tissus environnants. Ce problème de l'invasivité concerne en particulier l'introduction des implants dans le rachis et notamment l'accès aux espaces intervertébraux (espaces discaux) qui est souvent particulièrement délicat à cause de l'encombrement, par exemple du fait de la présence de vaisseaux sanguins et de nerfs aux abords de l'espace intervertébral, ainsi que de la proximité de la moelle épinière. Les implants, et leurs dispositifs d'ancrage osseux qui doivent pénétrer dans les vertèbres suffisamment profondément pour assurer une bonne fixation. Concernant l'implantation, diverses voies d'approche pour placer l'implant sont possibles, même si l'on préfère généralement une voie donnée pour chacun des divers étages rachidiens, parfois du fait de la préférence et de l'aisance du chirurgien pour cette voie, mais aussi de la pathologie à traitée (une hernie peut être plutôt postérieur, donc plus aisée à retirer par une voie postérieur), mais encore du fait de l'anatomie du patient (des vaisseaux dans l'axe de la voie d'abord antérieure, ou des muscles trop proéminents, etc.). On pourra privilégier par exemple (de manière non-limitative) un abord mini-invasif (MIS, pour l'anglais « Mini-Invasive Spine Surgery) antérieur médian pour les vertèbres cervicales et un abord mini-invasif latéral ou antéro-latéral pour les vertèbres thoraciques ou lombaires. En particulier, certains implants (notamment les cages intersomatiques), généralement au niveau lombaire sont prévus pour être implantés par une voie postérieure (depuis l'arrière du patient) ou une voie transforaminale (au travers du foramen). La voie postérieure nécessite généralement une résection (généralement partielle) des articulaires et/ou des facettes et passe entre la dure-mère et les articulaires (on prévoit en général deux cages disposées chacune d'un côté du plan sagittal). Cette voie emprunte donc un trajet très proche de la moelle épinière. La voie transforaminale emprunte une voie oblique par rapport au plan sagittal et nécessite des cages de longueur suffisante pour être disposées obliquement ou perpendiculairement au plan sagittal. On recherche généralement des voies d'accès les plus petites possible pour limiter l'invasivité de l'opération chirurgicale d'implantation. Ainsi, pour obtenir un implant le moins invasif possible (i.e., ne nécessitant pas le dégagement d'une large voie d'approche), il est nécessaire de réduire l'encombrement de l'implant et idéalement de limiter la taille du passage nécessaire à la fois à l'implantation et à la fixation de l'implant. En effet, les implants qui sont ancrés dans les vertèbres rajoutent souvent une contrainte supplémentaire sur l'encombrement et l'invasivité.

On notera que le problème d'invasivité fournit des contraintes supplémentaires pour répondre au problème de stabilité, notamment parce que le fait de réduire les dimensions pour réduire l'invasivité s'accompagne de risques d'instabilité. Il est donc intéressant de proposer une solution qui permette de concilier les contraintes liées à l'invasivité et à la stabilité.

Dans ce contexte, il est intéressant de proposer une solution permettant de répondre efficacement à au moins une partie de ces problèmes.

### DESCRIPTION GENERALE DE L'INVENTION

La présente invention a pour but de pallier certains inconvénients de l'art antérieur en proposant une combinaison d'implants osseux, en particulier destinés à une implantation par abord mini-invasif (MIS) dans l'espace discal entre les vertèbres adjacentes, permettant en outre une implantation stable, facile, rapide et avec une invasivité réduite.

Ce but est atteint par un système d'arthrodèse rachidienne comprenant plusieurs implants de plusieurs types, pour l'arthrodèse d'au moins deux vertèbres adjacentes, caractérisé en ce qu'il comporte, d'une part:
- au moins un implant intersomatique, destiné à être implanté dans l'espace discal entre lesdites vertèbres adjacentes pour maintenir une distance entre elles et comportant au moins un dispositif d'ancrage osseux pour ancrer ledit implant intersomatique dans chacune desdites vertèbres et les immobiliser l'une par rapport à l'autre,
et, d'autre part, au moins un implant parmi les deux types d'implants suivants :
- au moins un implant de type interépineux, destiné à être implanté entre deux épines vertébrales desdites vertèbres adjacentes, l'implant interépineux comportant au moins deux ailes de dimensions agencées pour être inséré entre les deux épines ,depuis une de leurs faces latérales ;
- au moins un implant de type facettaire, destiné à être implanté entre et/ou à travers les facettes articulaires des vertèbres et formé par un corps allongé selon un axe longitudinal et munie d'une tête et de spires d'au moins un filetage, sur au moins une portion à proximité de l'extrémité libre, ledit corps comportant au moins un conduit interne longitudinal , sur au moins une portion le long de l'axe longitudinal, et/ou des fenêtres traversant ledit corps transversalement à l'axe longitudinal et/ou des moyens de stabilisation au niveau de la tête pour prendre appui sur les tissu osseux environnants.

Ce type de solution, utilisant une combinaison de plusieurs implants de plusieurs types, qui sont peu invasifs et faciles à implanter, présente en outre l'avantage de fournir une arthrodèse plus étendue et plus fiable par effet cumulé et synergique des divers types d'implants utilisés. En effet, cette combinaison rendue possible par les caractéristiques techniques de faibles encombrement et invasivité des implants, permet d'immobiliser au moins deux des trois grands axes de stabilité du rachis, et ainsi d'obtenir une arthrodèse plus rapide et plus fiable qu'avec les implants utilisés indépendamment les uns des autres. Enfin, afin d'optimiser l'arthrodèse et réduire l'invasivité des implants, lesdits implants sont configurés pour être aptes à être implantés par une voie d'accès aux vertèbres la moins invasive, c'est-à-dire par une voie d'insertion postérieure ou transforaminale, au moins au niveau lombaire.

Selon une autre particularité, ledit système pour arthrodèse d'au moins deux vertèbres adjacentes, comprend au moins un implant intersomatique et au moins un implant interépineux.

Selon une autre mode de réalisation, ledit système pour arthrodèse d'au moins deux vertèbres adjacentes, comprend au moins un implant intersomatique et au moins un implant facettaire.

Selon une autre particularité, ledit système comprend un implant intersomatique postérieur. Un implant intersomatique postérieur signifiant que ledit implant est destiné à être insérer dans l'espace discale entre les deux vertèbres adjacentes, par un abord ou voie postérieur(e).

Selon une autre particularité, ledit système comprend un implant intersomatique transforaminal. Un implant intersomatique transforaminal signifiant que ledit implant est destiné à être insérer dans l'espace discale entre les deux vertèbres adjacentes, par un abord ou voie transforaminal(e).

Selon un autre mode de réalisation, ledit implant intersomatique présente un profil allongé et courbé pour affecter par exemple une forme de banane ou de demi-banane.

Selon une autre particularité, ledit implant intersomatique comporte au moins une paroi périphérique, dont au moins une partie, dite postérieure, comportant au moins un passage, dimensions et orientation complémentaires aux formes et dimensions dudit dispositif d'ancrage comportant au moins un corps rigide et allongé selon un axe longitudinal s'étendant entre une première extrémité, dite antérieure, et une seconde extrémité, dite postérieure, ledit corps étant inséré sans déformation dans ledit passage, sensiblement dans le plan de l'implant intersomatique, par coulissement depuis ladite partie postérieure de l'implant intersomatique, ledit passage traversant l'implant intersomatique depuis la périphérie vers une surface supérieure ou inférieure de sorte que l'extrémité antérieure de ledit corps pénètre dans une desdites vertèbres adjacentes, tandis que l'extrémité postérieure reste dans ledit passage et retient ledit implant intersomatique contre ladite vertèbre.

Selon une autre particularité, ledit corps du dispositif d'ancrage est courbe.

Selon une autre particularité, ledit corps du dispositif d'ancrage est courbe de sorte à pouvoir pénétrer une des vertèbres adjacentes en étant introduit dans l'implant intersomatique selon un axe d'approche sensiblement dans le même plan que celui de l'implant intersomatique.

Selon une autre particularité, au moins une des surfaces supérieure et inférieure de la paroi périphérique comporte des crans évitant le déplacement de l'implant intersomatique entre les vertèbres entre lesquelles il est destiné à être implanté.

Selon une autre particularité, ledit corps du dispositif d'ancrage comporte au moins une nervure ou seconde plaque coopérant avec au moins une rainure ménagée dans le passage de l'implant.

Selon une autre particularité, ladite extrémité postérieure de ledit corps du dispositif d'ancrage comporte au moins une butée pressant l'implant intersomatique contre ladite vertèbre.

Selon une autre particularité, ledit corps du dispositif d'ancrage comporte au moins une butée s'opposant au retrait dudit dispositif d'ancrage de l'implant intersomatique.

Selon une autre particularité, ladite extrémité postérieure de ledit corps du dispositif d'ancrage comporte au moins une butée orientée non parallèlement à l'axe longitudinal du corps et complémentaire d'au moins une butée d'au moins un moyen de verrouillage du dispositif par rapport à l'implant intersomatique , ledit moyen de verrouillage qui équipe l'implant étant muni d'au moins une portion flexible permettant, d'une part, de repousser ladite butée du moyen de verrouillage pour l'insertion du dispositif d'ancrage dans le passage, et d'autre part, l'engagement réciproque des deux butées lorsqu'elles se retrouvent l'une en face de l'autre, par le retour élastique de la portion flexible.

Selon une autre particularité, la paroi périphérique de l'implant intersomatique comporte au moins un moyen d'accrochage destiné à coopérer avec une extrémité de préhension d'une instrumentation d'implantation.

Selon une autre particularité, les plans moyens passant par les surfaces supérieure et inférieure de l'implant intersomatique forment un angle orienté selon un axe antéropostérieur de l'implant intersomatique permettant d'imposer une lordose ou une cyphose aux vertèbres entre lesquelles l'implant intersomatique est destiné à être implanté.

Selon une autre particularité, les plans moyens passant par les surfaces supérieure et inférieure de l'implant intersomatique sont sensiblement parallèles entre eux.

Selon une autre particularité, la paroi périphérique comporte deux passages orientés chacun vers une des surfaces supérieure et inférieure, de façon à permettre l'ancrage du dispositif d'ancrage dans chacune des vertèbres adjacentes entre lesquelles l'implant intersomatique est destiné à être implanté.

Selon une autre particularité, la paroi périphérique comporte au moins un chanfrein sur au moins une portion périphérique d'au moins une de ses surfaces supérieure et inférieure, de façon à faciliter l'insertion de l'implant intersomatique entre les vertèbres.

Selon une autre particularité, ledit implant interépineux comprend des moyens d'immobilisation d'au moins une des épines (El, ES) entre lesquelles il est destiné à être implanté.

Selon une autre particularité, lesdits moyens d'immobilisation sont des moyens de fixation ou d'ancrage dans au moins une des épines (El, ES).

Selon une autre particularité, lesdits moyens d'immobilisation sont des moyens de crochetage d'au moins une des épines (El, ES).

Selon une autre particularité, lesdits moyens d'immobilisation sont des moyens de compression d'au moins une des épines (El, ES).

Selon une autre particularité, l'implant interépineux comporte deux ailes disposées chacune sur une face latérale de l'implant opposée à l'autre aile et projetant chacune vers une des deux épines, de sorte que les ailes longent chacune une épine mais sur des faces latérales (E4, E5) opposées, l'insert étant de forme sensiblement sigmoïdale par le fait que son aile comporte au moins deux rayons de courbures d'orientations opposées, de sorte que deux faces de l'aile comportent chacune une portion concave et une portion convexe, le passage et l'insert étant agencés de sorte que, lorsque l'insert est logé dans le passage, au moins une partie desdites portions convexes des deux faces de l'insert longent chacune au moins une portion des épines, sur des faces latérales opposées à celles longées par les ailes.

Selon une autre particularité, lesdits moyens de compression pour comprimer les faces latérales des épineuses entre lesdites ailes et ledit insert lorsque ce dernier est inséré au travers dudit passage de l'implant interépineux.

Selon une autre particularité, les moyens de compression sont formés par deux ailes courbes de l'insert reliées par un axe d'articulation permettant le déploiement des deux ailes depuis une position repliée lors de l'insertion de l'insert dans le passage à une position déployée où lesdites ailes compriment les épineuses contre les ailes de l'implant.

Selon une autre particularité, l'insert de l'implant interépineux est retenu dans le corps par au moins un mécanisme de butée.

Selon une autre particularité, l'implant interépineux comporte, du côté opposé à celui muni des deux ailes, au moins un chanfrein facilitant l'insertion de l'implant entre les deux épines adjacentes.

Selon une autre particularité, l'implant interépineux comporte au moins un moyen de crochetage d'épineuse agencé pour aller se crocheter autour d'au moins une partie des bords des épines qui sont opposés aux bords des épines entre lesquelles le corps de l'implant est inséré.

Selon une autre particularité, ledit conduit interne de l'implant facettaire est obtenu par au moins un premier usinage central parallèle à l'axe longitudinal et les dites fenêtres de l'implant facettaire sont obtenues par au moins un second usinage dans un plan, dit transverse, non parallèle à l'axe longitudinal, de sorte que lesdites fenêtres préservent au moins une partie desdites spires et la paroi du corps derrière les spires, et préservent des portions non usinées sur le pourtour dudit corps de l'implant facettaire.

Selon une autre particularité, lesdites fenêtres de l'implant facettaire comportent au moins un bord latéral extérieur affuté.

Selon une autre particularité, ladite extrémité libre du corps de l'implant facettaire est auto-foreuse.

Selon une autre particularité, au moins une partie dudit corps de l'implant facettaire est sensiblement cylindrique ou conique ou tronconique.

Selon une autre particularité, le pourtour dudit filetage est sensiblement cylindrique malgré la forme cylindrique ou conique ou tronconique du corps de l'implant facettaire.

Selon une autre particularité, lesdites fenêtres sont alignées entre elles le long de l'axe longitudinal.

Selon une autre particularité, lesdites fenêtres sont décalées les unes par rapport aux autres le long de l'axe longitudinal.

Selon une autre particularité, ladite tête de l'implant ferme le conduit interne longitudinal ou comporte des moyens de fermeture du conduit interne longitudinal.

Selon une autre particularité, ledit filet possède un pas variable se raccourcissant en direction de la tête.

Selon une autre particularité, ledit corps est muni de plusieurs filets de pas différents, le pas d'un filet situé du côté de l'extrémité libre étant de taille plus important que le filet adjacent situé du côté de la tête.

Selon une autre particularité, ladite tête de l'implant facettaire est munie de moyens de stabilisation de l'implant, destinés à prendre appui sur le tissu osseux autour de ladite tête.

Selon une autre particularité, lesdits moyens de stabilisation comportent au moins un élément de stabilisation comprenant au moins deux tiges ayant une extrémité libre pointue et sensiblement parallèles à l'axe longitudinal et aptes à pénétrer le tissu autour de la tête et éventuellement d'une portion dudit corps de l'implant facettaire à proximité de ladite tête.

Selon une autre particularité, lesdites tiges sont reliées entre elles par une bague rendant l'élément de stabilisation apte à être monté sur ladite tête.

Selon une autre particularité, lesdits moyens de stabilisation comportent au moins un élément de stabilisation en forme de cloche montée sur la tête et dont le pourtour est destiné à prendre appui sur le tissu osseux entourant la tête.

Selon une autre particularité, ladite cloche comporte au moins une pointe ou dent sur son pourtour pour faciliter l'ancrage osseux.

Selon une autre particularité, ladite cloche est montée solidaire de la tête.

Selon une autre particularité, ladite tête possède une surface inférieure périphérique en forme de portion de sphère et complémentaire d'une surface supérieure interne de ladite cloche ainsi articulée sur la tête de l'implant.

Selon une autre particularité, lesdits moyens de stabilisation comportent des moyens de verrouillage appuyant sur l'élément de stabilisation pour le maintenir appuyé contre le tissu osseux.

Selon une autre particularité, au moins une partie desdites fenêtres sont séparées par au moins deux spires dépourvues de fenêtres.

Selon une autre particularité, au moins une partie desdites fenêtres sont ménagées sur plusieurs spires.

D'autres particularités et avantage de la présente invention sont détaillés dans la description qui suit.

### DESCRIPTION DES FIGURES ILLUSTRATIVES

D'autres particularités et avantages de la présente invention apparaîtront plus clairement à la lecture de la description ci-après, faite en référence aux dessins annexés, dans lesquels :

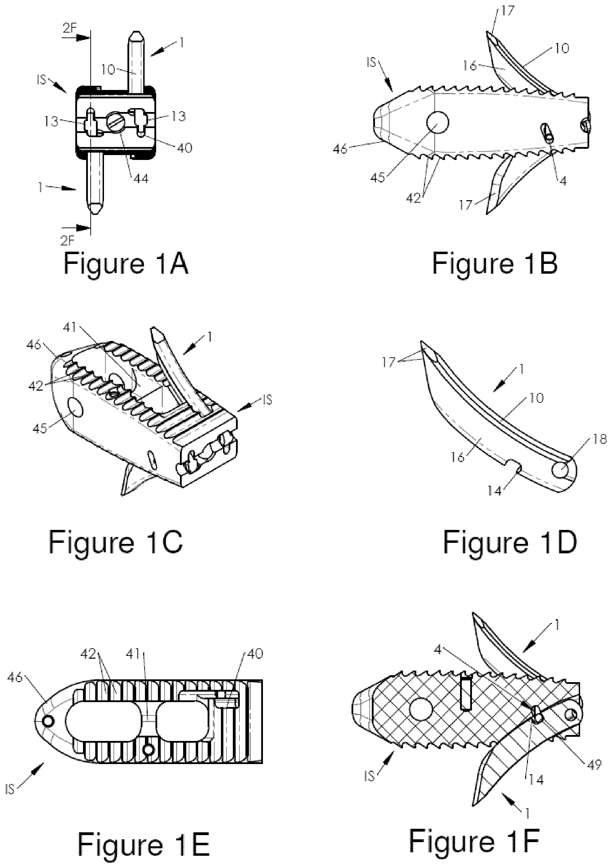

- les figures 1A, 1B, 1C et 1F représentent des vues, respectivement, de l'arrière, de profil, en perspective et en coupe selon le plan 2F-2F de la figure 1A, d'un implant intersomatique muni d'un dispositif d'ancrage de type ancre selon un mode de réalisation, la figure 1D représente une vue de profil du même dispositif d'ancrage et la figure 1E représente une vue de dessus du même implant intersomatique ;

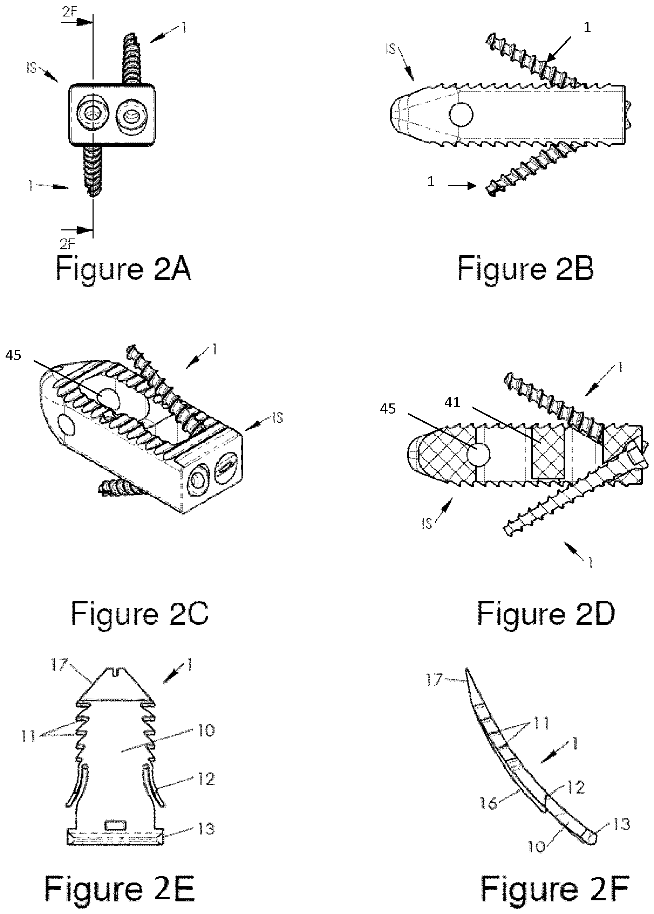

- les figures 2A, 2B, 2C et 2F représentent des vues, respectivement, de l'arrière, de profil, en perspective et en coupe selon le plan 2F-2F de la figure 2A, d'un implant intersomatique muni d'un dispositif d'ancrage type vis selon un mode de réalisation, les figures 2E et 2D représente une vue de profil d'un dispositif d'ancrage de type ancre d'un implant intersomatique selon un autre mode de réalisation ;

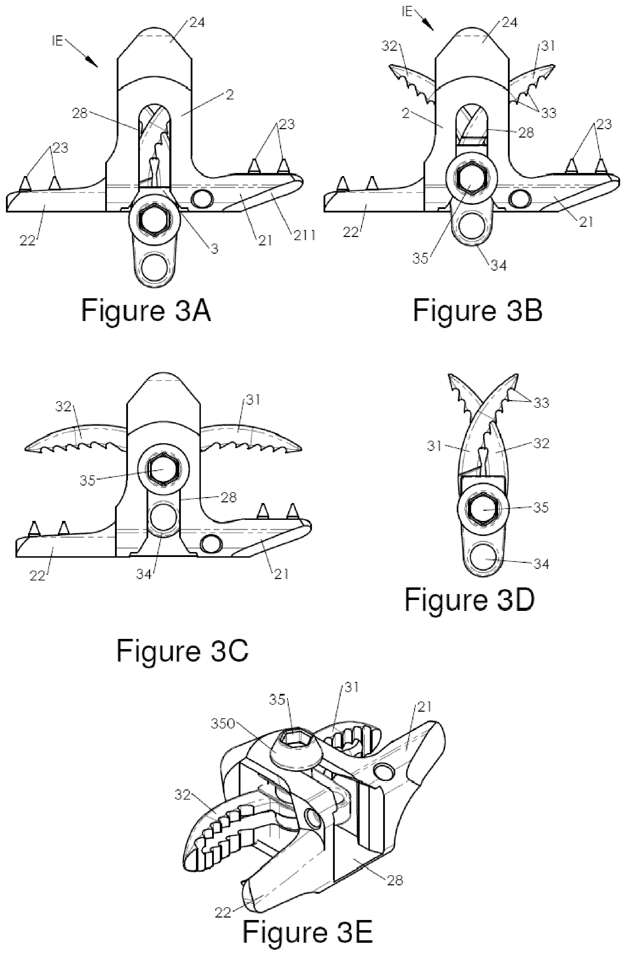

- les figures 3A, 3B et 3C représentent des vues de dessus d'un insert pour un implant interépineux selon un mode de réalisation, respectivement, pendant l'insertion, pendant le déploiement et après déploiement d'un insert déployable dans l'implant interépineux, la figure 3D représente une vue de dessus d'un insert pour implant interépineux, en position repliée et la figure 3E représente une vue en perspective d'un implant interépineux muni de son insert déployé ;

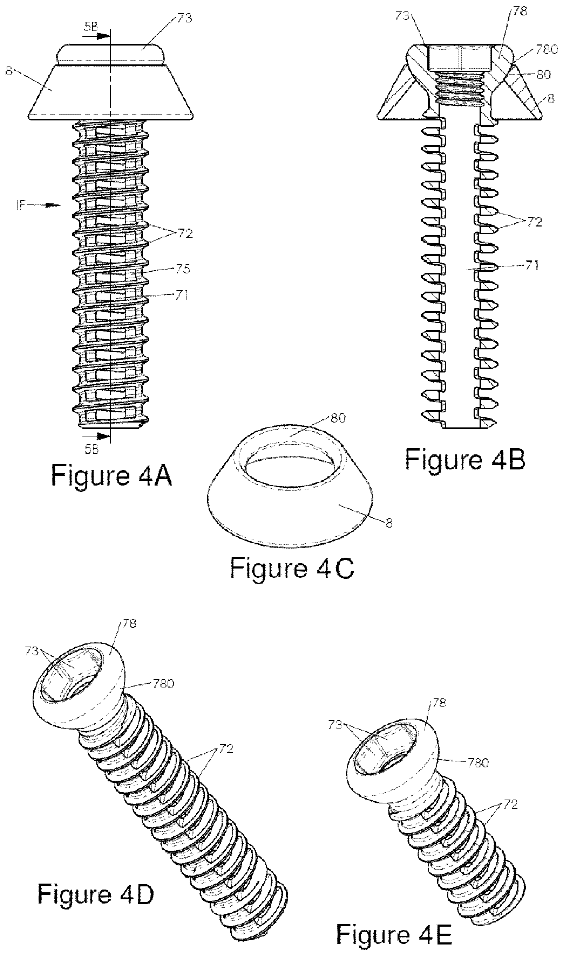

- les figures 4A représentent une vue de profil d'un implant facettaire selon une mode de réalisation, la figure 4B représente une vue en coupe selon le plan 5B-5B de la figure 4A et les figures 4C, 4D et 4E représentent des vues en perspective, respectivement, d'un élément de stabilisation et de deux variantes d'un implant facettaire selon un mode de réalisation ;

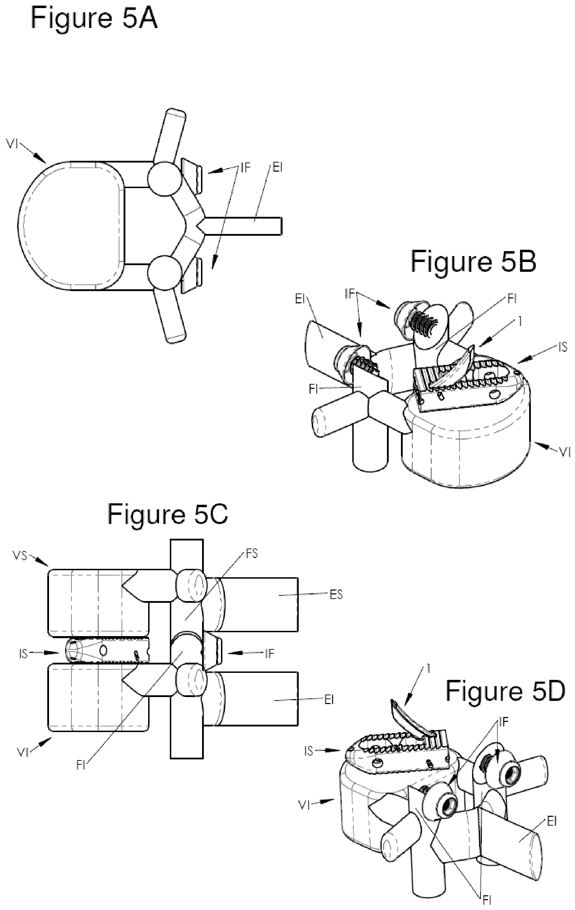

- les figures 5A, 5B, 5C et 5D représentent des vues, respectivement, de dessous, en perspective avant de profil et en perspective arrière d'un système d'arthrodèse selon un mode de réalisation comprenant un implant intersomatique et deux implants facettaires pour l'arthrodèse de deux vertèbres adjacentes dont au moins une est visible sur chacune de ces figures ;

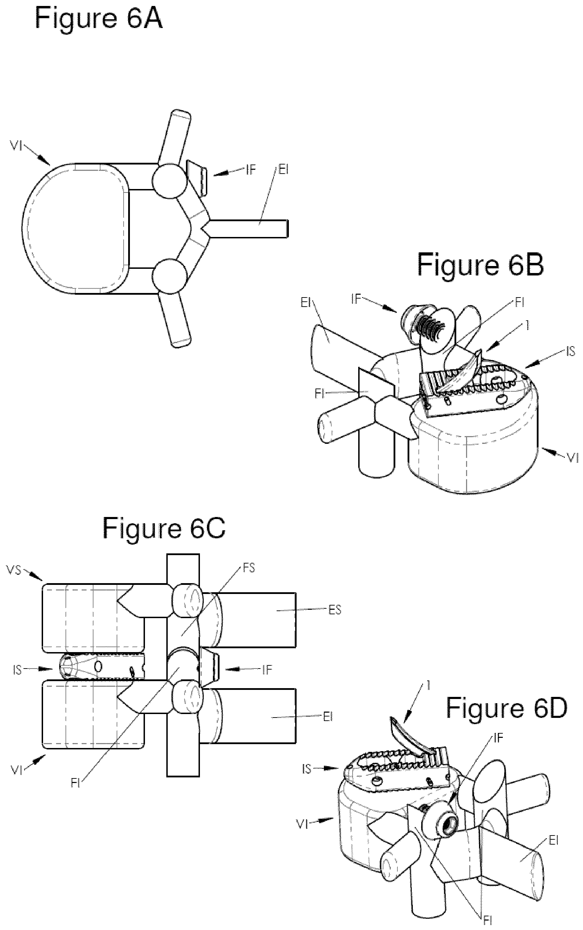

- les figures 6A, 6B, 6C et 6D représentent des vues, respectivement, de dessous, en perspective avant, de profil et en perspective arrière, d'un système d'arthrodèse selon un mode de réalisation comprenant un implant intersomatique et un implant facettaire pour l'arthrodèse de deux vertèbres adjacentes dont au moins une est visible sur chacune de ces figures;

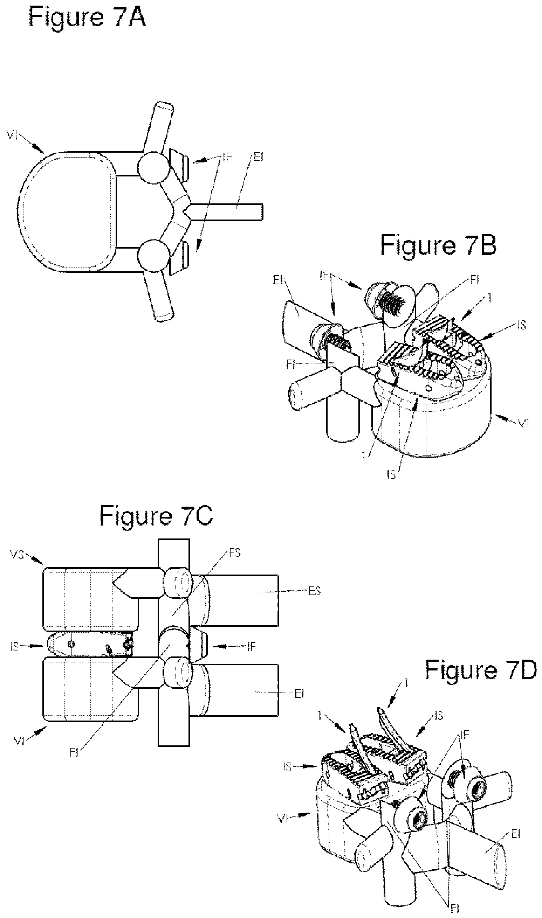

- les figures 7A, 7B, 7C et 7D représentent des vues, respectivement, de dessous, en perspective avant, de profil et en perspective arrière, d'un système d'arthrodèse selon un mode de réalisation comprenant deux implants intersomatiques et deux implants facettaires pour l'arthrodèse de deux vertèbres adjacentes dont au moins une est visible sur chacune de ces figures ;

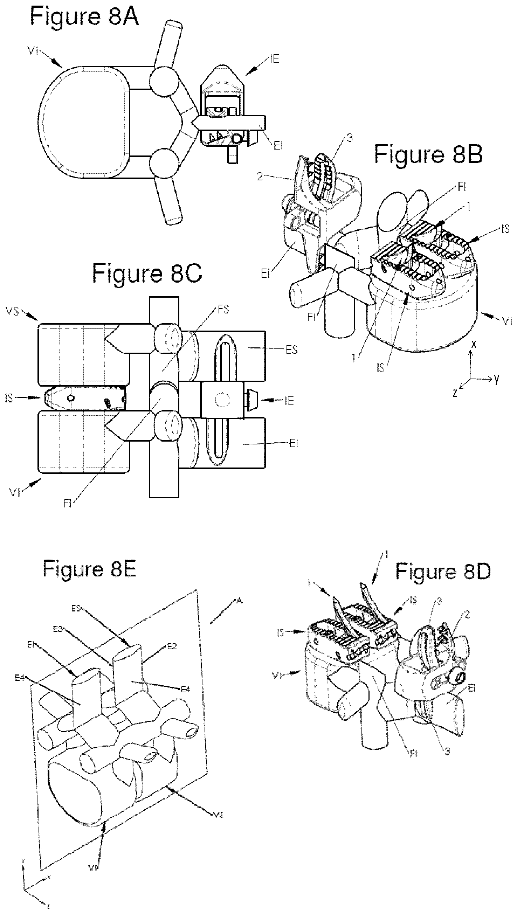

- les figures 8A, 8B, 8C et 8D représentent des vues, respectivement, de dessous, en perspective avant, de profil, en perspective arrière et de dessus d'un système d'arthrodèse selon un de mode de réalisation, comprenant deux implants intersomatique et un implant interépineux, pour l'arthrodèse de deux vertèbres adjacentes dont au moins une est visible sur chacune de ces figures, la figure 8E représente une vue en perspective orientée vers des faces latérales opposées de deux vertèbres adjacentes du rachis, illustrant les épines vertébrales, le plan sagittal du rachis et un repère tridimensionnel utilisé comme référence ;

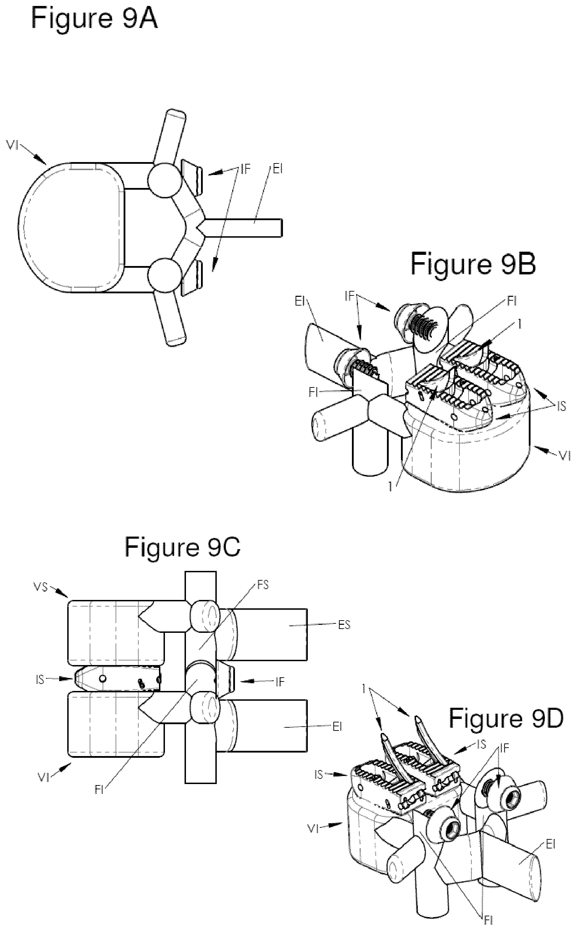

- les figures 9A, 9B, 9C et 9D représentent des vues, respectivement, de dessous, en perspective avant, de profil et en perspective arrière, d'un système d'arthrodèse selon un mode de réalisation comprenant deux implants intersomatiques et deux implants facettaires pour l'arthrodèse de deux vertèbres adjacentes dont au moins une est visible sur chacune de ces figures;

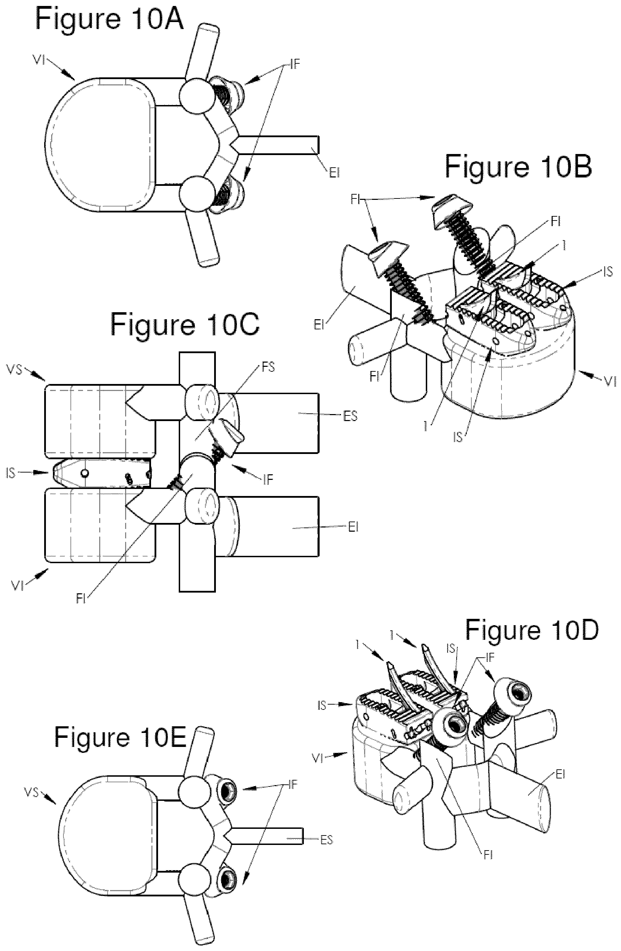

- les figures 10A, 10B, 10C, 10D et 10E représentent des vues, respectivement, de dessous, en perspective avant, de profil, en perspective arrière et de dessus, d'un système d'arthrodèse selon un mode de réalisation comprenant deux implants intersomatiques et deux implants transfacettaires" pour l'arthrodèse de deux vertèbres adjacentes dont au moins une est visible sur chacune de ces figures ;

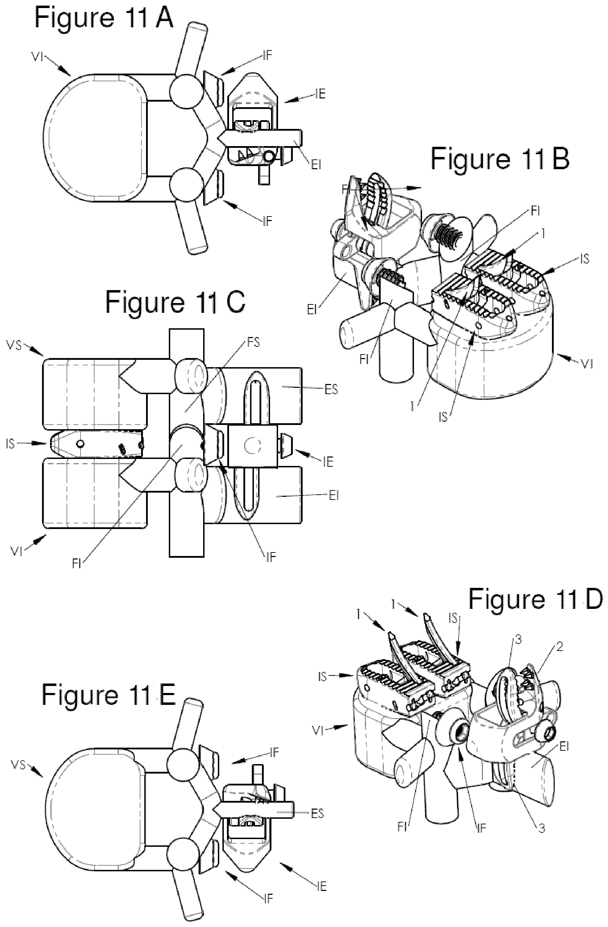

- les figures 11A, 11B, 11C, 11D et 11E représentent des vues, respectivement, de dessous, en perspective avant, de profil, en perspective arrière et de dessus d'un système d'arthrodèse selon un mode de réalisation comprenant un implant intersomatique, un implant interépineux et deux implants facettaires pour l'arthrodèse de deux vertèbres adjacentes dont au moins une est visible sur chacune de ces figures ;

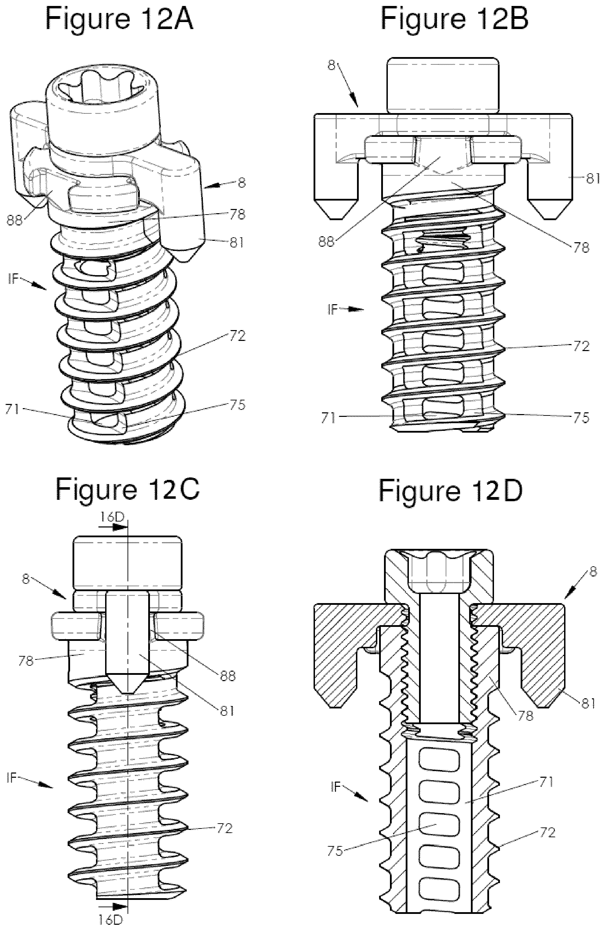

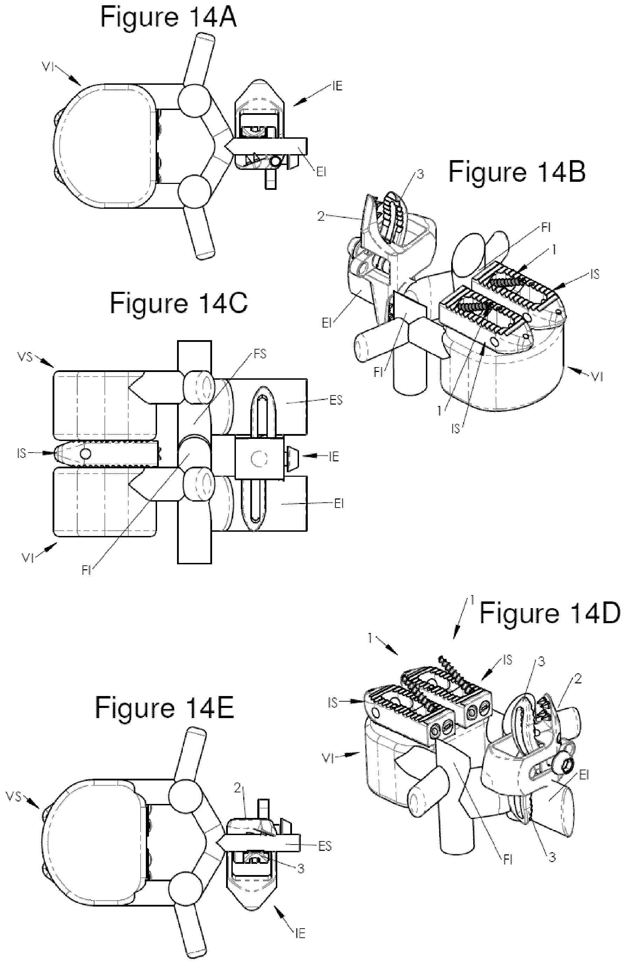

- les figures 12A, 12B et 12C représentent des vues, respectivement, en perspective, de face et de profil d'un implant facettaire selon un autre mode de réalisation, et la figure 12D représente une vue en coupe selon 16D-16D de la figure 14C du même implant;

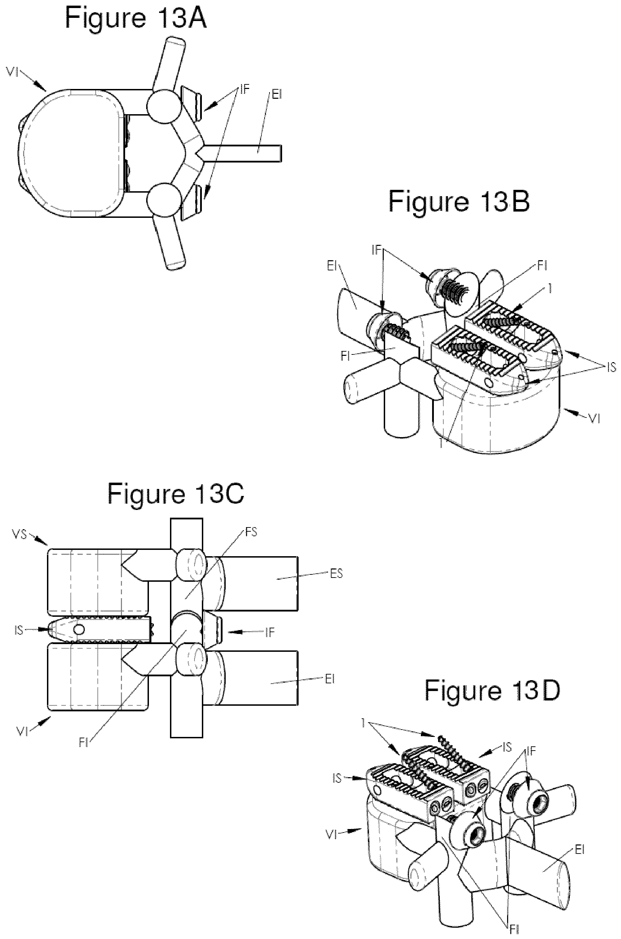

- les figures 13A, 13B, 13C et 13D représentent des vues, respectivement, de dessous, en perspective avant, de profil et en perspective arrière d'un système d'arthrodèse selon un mode de réalisation comprenant un implant intersomatique et deux implants facettaires, pour l'arthrodèse de deux vertèbres adjacentes dont au moins une est visible sur chacune de ces figures ;
- les figures 14A, 14B, 14C, 14D et 14E représentent des vues, respectivement, de dessous, en perspective avant, de profil, en perspective arrière et de dessus d'un système d'arthrodèse selon un mode de réalisation comprenant un implant intersomatique et un implant interépineux, pour l'arthrodèse de deux vertèbres adjacentes dont au moins une est visible sur chacune de ces figures.

### DESCRIPTION DES MODES DE REALISATION PREFERES DE L'INVENTION

La présente invention concerne un système pour l'arthrodèse (fusion osseuse) d'au moins deux vertèbres adjacentes. Un tel système comporte au moins deux implants d'au moins deux types différents. La présente invention concerne ainsi par exemple un système d'arthrodèse rachidienne comportant au moins deux types d'implants parmi les trois types suivants :
- Un implant intersomatique (IS),
- Un implant interépineux (IE),
- Un implant facettaire (IF).

Plus particulièrement, on choisira en général un système dont les implants présentent des caractéristiques techniques permettant de répondre à au moins une partie des problèmes évoqués dans la présente demande.

Ainsi, divers modes de réalisation concernent un système d'arthrodèse rachidienne comprenant plusieurs implants de plusieurs types, pour l'arthrodèse d'au moins deux vertèbres (VI, VS) adjacentes, caractérisé en ce qu'il comporte, d'une part:
- au moins un implant intersomatique (IS), destiné à être implanté dans l'espace discal entre lesdites vertèbres (VI, VS) adjacentes pour maintenir une distance entre elles et comportant au moins un dispositif d'ancrage osseux pour ancrer ledit implant intersomatique (IS) dans au moins une desdites vertèbres (VI, VS)
et, d'autre part, au moins un implant parmi les deux types d'implants suivants :
- au moins un implant de type interépineux (IE), comprenant un corps (2) de dimensions configurées pour maintenir un écartement entre deux épines vertébrales (El, ES) desdites vertèbres (VI, VS) adjacentes, l'implant interépineux (IE) comportant au moins deux ailes (21, 22, 31, 33) aptes à longer, chacune, au moins une portion d'au moins une face latérale d'au moins une desdites épines (E1, ES) ;
- au moins un implant de type facettaire (IF), allongé selon un axe longitudinal entre une extrémité libre et une tête (78) et destiné à être implanté entre et/ou à travers les facettes articulaires des vertèbres (VI, VS), ledit implant de type facettaire (IF) comprenant des moyens de stabilisation au niveau de la tête (78) pour prendre appui sur les tissu osseux environnants et/ou au moins une cale munie d'au moins une fixation osseuse ou seulement cette fixation osseuse, ladite cale et/ou ladite fixation osseuse comportant au moins un conduit interne longitudinal (71) dans au moins une portion le long de l'axe longitudinal, et/ou des fenêtres (75) traversant ladite cale et/ou ladite fixation osseuse transversalement à l'axe longitudinal.

De préférence, ladite fixation osseuse de l'implant facettaire (IF) est:
- soit formée par une ancre rigide, de préférence courbe, par exemple similaire au dispositif d'ancrage osseux (1) de l'implant intersomatique (IS), mais avec des dimensions réduites pour une utilisation dans les facettes articulaires;
- soit formée par une vis munie de spires (72) d'au moins un filetage, sur au moins une portion à proximité de l'extrémité libre.

De plus, ledit implant interépineux (IE) comprend en général des moyens d'immobilisation d'au moins une des épines (El, ES) entre lesquelles il est destiné à être implanté. Divers exemples et avantages de tels moyens d'immobilisation sont décrits ci-après.

De préférence, ledit implant intersomatique (IS) est configuré pour une implantation dans l'espace discal entre lesdites vertèbres (VI, VS) adjacentes, par un abord mini-invasif (MIS), par exemple en passant par la même incision que l'autre type d'implant (ou via de petites incisions proches les unes des autres, mais de préférence en limitant les lésions restreintes des tissus sous-jacents lors du passage des implants). En particulier, divers modes de réalisation vise à utiliser une même voie d'abord (d'approche) pour l'implantation des divers implants du système ou du moins une implantation dans un même temps chirurgical, c'est-à-dire que le patient est placé dans une position qu'il n'est pas nécessaire de modifier pour l'insertion d'un implant à l'autre du système (de préférence sur le ventre, selon les modes de réalisation préférés). Ainsi, le chirurgien peut par exemple insérer au moins un implant intersomatique (IS) d'immobilisation d'au moins deux vertèbres adjacentes et une stabilisation additionnelle, de type implant facettaire (IF) ou implant inter-épineux (IE) par différents abords de tailles limitées (quelques centimètres au maximum) et proches les uns des autres (éloignés de seulement quelques centimètres). De plus, on préfère en général des implants avec un faible encombrement pour éviter de mettre en danger les vaisseaux sanguins et les tissus nerveux environnants, et on préfère souvent que les implants soient ancrés à l'aide de dispositifs d'ancrage osseux qui doivent pénétrer dans les vertèbres suffisamment profondément pour assurer une bonne fixation et permettre d'obtenir un implant stable. Un implant peu invasif sera par exemple défini par le fait qu'il (et/ou que sa fixation) ne nécessite pas plus de place (ou peu) aux abords de l'espace intervertébral que la place nécessaire pour l'introduction de l'implant rachidien lui-même.

On préfère généralement des voies d'accès les plus petites possibles pour limiter l'invasivité de l'opération chirurgicale d'implantation. Donc, pour obtenir un implant le moins invasif possible ne nécessitant par le dégagement d'une large voie d'approche, il est nécessaire de réduire l'encombrement de l'implant et de limiter la taille du passage pour l'implantation et la fixation. Ainsi, on préfère en général un implant intersomatique (IS) configuré pour une implantation depuis la face arrière de la colonne vertébrale, c'est-à-dire selon les approches dorsale (ou postérieure) ou transforaminale bien connues de l'homme de métier.

Ainsi, avec au moins un tel implant intersomatique (IS) combiné à au moins un implant interépineux (IE) et/ou au moins un implant facettaire (IF), il est possible de fournir un système peu invasif offrant une stabilisation vertébrale en plusieurs points et permettant une arthrodèse rapide et fiable, tout en réduisant la durée de l'opération nécessaire à l'implantation. En effet, un tel système permet de surmonter l'incompatibilité qui existait jusqu'alors entre, d'une part, le fait d'implanter plusieurs implants au cours d'une intervention chirurgicale et, d'autre part, le fait de rester le moins invasif possible et le plus rapide possible. De plus, un tel système immobilise les vertèbres en plusieurs points et permet d'éviter que la fusion osseuse ne se produise dans une position relative des vertèbres qui n'est pas celle souhaitée lors de l'implantation. En effet, à cause des mouvements des patients pendant que l'arthrodèse est en cours, il existe un risque que les vertèbres se déplacent légèrement l'une par rapport à l'autre, résultant à une fusion osseuse dans une position non souhaitée ou même à une non fusion. C'est pour cette raison qu'il est souvent fait usage dans l'art antérieur de barres de stabilisation fixées par des vis pédiculaires, alors qu'elles sont très invasives et difficiles à implanter, contrairement aux divers modes de réalisation de la présente invention. Divers modes de réalisation de l'invention vise donc à fournir une arthrodèse plus fiable grâce à une immobilisation des vertèbres plus efficace grâce à une maintien des vertèbres sur au moins deux zones (généralement entre deux et cinq zones).

L'utilisation d'implants intersomatiques ancrés dans les vertèbres et combinés à au moins un autre implant situé de l'autre côté du canal médullaire fournit donc un système équivalent aux solutions invasives connues, en termes de stabilité, tout en offrant les nombreux avantages détaillés ci-dessus. Les divers modes de réalisation du système permettent donc une stabilisation en deux ou trois points (corps vertébral, facettes, épine) ce qui permet une bonne répartition des charges et des forces, en plus de permettre, à terme, une fusion en un ou deux points (corps vertébral, facettes), voire en trois points mais la fusion des épines n'est généralement pas prévue. De plus, le fait d'utiliser plusieurs zones de maintien de la position des vertèbres permet de compenser d'éventuels défauts de morphologie des vertèbres, au niveau des corps vertébraux, des facettes et/ou des épineuses. D'autre part, cette utilisation combinée d'au moins deux implants permet de mieux contrôler les corrections de la courbure du rachis qu'il est parfois souhaitable de réaliser, grâce au fait que le système impose une position relative des vertèbres en agissant sur plusieurs zones de ces vertèbres qui se trouvent ainsi maintenues dans cette position relative de manière plus fiable et/ou plus efficace. Dans les divers modes de réalisation présentés dans la présente demande, on préfère donc un implant intersomatique de type "postérieur" ou "transforaminal' (ces implants étant souvent appelés cages intersomatiques ou intervertébrales ou cages d'arthrodèse). L'homme de métier, notamment les chirurgiens connaissent bien les divers types d'implants intersomatiques et apprécieront les caractéristiques techniques qui découlent de ces termes, parmi lesquelles on peut citer, à titre d'exemple non limitatif, une forme relativement allongée (pour les postérieures), parfois incurvée (pour les transforaminales). Ainsi, dans divers modes de réalisation préférés de l'invention, l'implant intersomatique (IS) présente des dimensions le rendant apte à une des approches postérieure ou transforaminale. Pour un implant intersomatique(IS) lombaire, ces deux approches seront préférées grâce à la synergie des implants (IS, IE, IF) combinés dans le système, comme détaillé ci-dessus. Par exemple, pour un implant postérieur, la hauteur de l'implant (i.e., sa dimension selon l'axe vertical du rachis une fois implanté) pourra, au niveau postérieur (i.e., vers la face dorsale du patient) être de l'ordre de 5 mm, mais pourra aller jusqu'à une valeur d'environ 20mm, généralement moins de 10 à 16 mm. La hauteur au niveau antérieur (i.e., vers la face ventrale du patient) sera généralement comprise entre 10 mm et 16 mm mais pourra aller jusqu'à une valeur d'environ 20mm. Ces dimensions sont généralement ajustées en fonction de l'étage vertébral et de la pathologie et sont ajustées en fonction l'une de l'autre pour régler la lordose fournie par l'implant. La largeur de l'implant intersomatique (IS) postérieur sera généralement sensiblement constante sur toute sa profondeur (ou longueur), même s'il est souvent prévu de légères variations de formes pour rendre l'implant plus fuselé, par exemple pour offrir une meilleure pénétration. Cette largeur au moment de l'insertion de l'implant dans son passage pour un passage de l'implant par la voie postérieure sera inférieure à 20 mm et de préférence en général inférieur à 15mm pour éviter de léser la moelle épinière et/ou les racines nerveuses. Enfin, la profondeur (ou longueur, mais le terme profondeur est plus adapté puisqu'on désigne ici la dimension dans laquelle on enfonce l'implant dans l'espace discal) sera en général comprise entre 18 mm et 38 mm, de préférence entre 24mm et 27mm. En ce qui concerne l'implant intersomatique(IS) transforaminal, préféré pour une fusion intervertébrale lombaire transforaminale (TLIF), la largeur sera généralement aussi sensiblement constante sur toute sa profondeur (ou longueur), même s'il est souvent prévu de légères variations de formes et surtout une fréquente incurvation de l'implant dans cette direction, de façon à ce qu'il puisse être positionné de façon optimale dans l'espace discal. Cette largeur sera également inférieure à 15mm mais ce type d'implant peut parfois être plus large car l'abord aux vertèbres selon cette voie est parfois moins délicat que par la voie postérieure. De même que pour les implants postérieurs, la hauteur ou les hauteurs de l'implant transforaminal, aux niveaux antérieur et postérieur, sera (ou seront) choisies en fonction de la configuration des vertèbres et de l'éventuelle lordose souhaitée, dans les mêmes valeurs inférieure à 20 mm, avec la différence particulière que l'orientation oblique de l'implant impose que l'angle formé entre les surfaces supérieur et inférieur soit réalisé de manière adéquate pour l'orientation finale de l'implant dans l'espace discal. En revanche, cette orientation oblique permet que ce type d'implant transforaminal soit plus long que l'implant postérieur et présente donc une profondeur comprise entre 25mm et 40mm, de préférence entre 30mm et 35mm.

L'homme de métier comprendra donc aisément qu'il pourra implanter facilement et rapidement ce type d'implant intersomatique (patient en decubitus ventral, sans avoir besoin de le retourner durant l'intervention) en même temps qu'au moins un implant facettaire et/ou un implant interépineux, en limitant les lésions du patient et il jugera facilement de la localisation et de l'étendue (restreinte) de l'incision (ou des incisions) qu'il pourra réaliser pour une telle implantation combinée du système.

Dans certains modes de réalisation, le système d'arthrodèse rachidienne, comprenant plusieurs implants de plusieurs types, pour l'arthrodèse d'au moins deux vertèbres (VI, VS) adjacentes, est caractérisé en ce qu'il comporte, d'une part:
- au moins un implant intersomatique (IS) transforaminal ou postérieur, destiné à être implanté dans l'espace discal entre lesdites vertèbres (VI, VS) adjacentes pour maintenir une distance entre elles et comportant au moins un dispositif d'ancrage osseux (1) pour ancrer ledit implant intersomatique (IS) dans au moins une desdites vertèbres (VI, VS)
et, d'autre part, au moins un implant parmi les deux types d'implants suivants :
- au moins un implant de type interépineux (IE), destiné à être implanté entre deux épines vertébrales (EI,ES) desdites vertèbres (VI, VS) adjacentes, l'implant interépineux (IE) comportant au moins deux ailes et, de préférence, des moyens d'immobilisation des épines vertébrales (EI,ES) ;
- au moins un implant de type facettaire (IF), allongé selon un axe longitudinal entre une extrémité libre et une tête (78) et destiné à être implanté entre et/ou à travers les facettes articulaires des vertèbres (VI, VS), ledit implant de type facettaire (IF) comprenant au moins une cale munie d'une fixation osseuse ou seulement au moins une fixation osseuse , , ladite cale et/ou ladite fixation osseuse comportant au moins un conduit interne longitudinal (71) dans au moins une portion le long de l'axe longitudinal, et/ou des fenêtres (75) traversant ladite cale et/ou ladite fixation osseuse transversalement à l'axe longitudinal et/ou des moyens de stabilisation au niveau de la tête (78) pour prendre appui sur les tissu osseux environnants. Selon divers modes de réalisation, lesdits moyens d'immobilisation sont des moyens de fixation ou d'ancrage dans au moins une des épines vertébrales (EI,ES) et/ou des moyens de crochetage d'au moins une des épines vertébrales (EI,ES) et/ou des moyens de compression d'au moins une des épines vertébrales (EI,ES).

De préférence, ladite fixation osseuse de l'implant facettaire (IF) dans ce système utilisant au moins un implant intersomatique (IS) postérieur ou transforaminal est:
- soit formée par une ancre rigide, de préférence courbe, par exemple similaire au dispositif d'ancrage osseux (1) de l'implant intersomatique (IS), mais avec des dimensions réduites pour une utilisation dans les facettes articulaires;
- soit formée par une vis munie de spires (72) d'au moins un filetage, sur au moins une portion à proximité de l'extrémité libre.
- En général, les implants facettaires sont des cales ou des vis et dans la présente demande, on tire avantage de la fixation osseuse par la vis (seule ou associée à une cale). La présente demande décrit donc préférentiellement les vis facettaires, avec un corps (80) fileté et une tête (78), mais on comprend que cet exemple ne doit pas être considéré comme étant limitatif.

Un objectif principal de la présente invention est de réaliser une fusion osseuse stable et rapide, tout en limitant l'invasivité de l'implantation. La stabilité de la fusion est assurée par l'utilisation d'un système comprenant une combinaison d'au moins deux types d'implants parmi les différents types d'implants intersomatique (IS), interépineux (IE) et facettaire (IF) décrits dans la présente demande. Les différents types d'implant du système sont configurés pour une implantation dans l'espace discal entre lesdites vertèbres (VI, VS) par un abord mini-invasif (MIS). L'implantation par abord MIS permet d'insérer un ou plusieurs desdits implants par une approche chirurgicale lésant le moins possible les tissus du patient, grâce à une réduction du nombre d'incision et/ou de la taille de l'incision et/ou de l'étendue des ablations ou résections par exemple. Ainsi, ledit système d'arthrodèse présente l'avantage d'utiliser une combinaison d'implants choisis pour leurs stabilités et pour les effets de synergie possibles entre eux puisque leur utilisation combinée permet d'obtenir une arthrodèse de stabilité supérieure à celle obtenue par chaque implant utilisé seul. De plus, le choix des implants combinés dans les divers modes de réalisation du système permet d'obtenir, d'une part, une invasivité inférieure à celle d'autres combinaisons d'implants et, d'autre part, une implantation plus facile et rapide qu'avec d'autres combinaisons d'implants. En effet, ces synergies sont en particulier obtenues dans divers modes de réalisation de l'invention en imposant les caractéristiques d'implantation par voie postérieure ou transforaminale pour l'implant intersomatique, de préférence muni d'un ancrage vertébral, en combinaison avec un implant interépineux à ailes déployables qui peut donc être inséré entre les épines depuis une seule de leurs faces latérales et/ou avec un implant facettaire qui est généralement rapidement vissé entre ou au travers des facettes articulaires. Ainsi, par un abord depuis la face dorsale du patient, le long des épineuses, éventuellement au travers d'une seule incision (ou d'une seule incision de chaque coté des épineuses), le chirurgien pourra réaliser une arthrodèse avec tous les avantages détaillés ci-dessus.

### Les différentes configurations du système d'arthrodèse :

Dans certains modes de réalisation, le système comporte au moins un implant de type intersomatique (IS), de préférence ancré dans au moins une desdites vertèbres (VI, VS), mais idéalement ancré dans ces deux vertèbres (VI, VS) adjacentes. De plus, cet implant intersomatique (IS) est de préférence combiné à un implant de type interépineux (IE) et/ou à au moins un implant facettaire (IF). On notera que l'expression "au moins un " utilisée dans la présente demande pour les implants intersomatique (IS) et facettaire (IF) désigne en fait souvent seulement les alternatives d'un seul implant ou de deux implants, car il est rare que l'on utilise plus que deux exemplaires de ces implants puisque l'on n'en implante rarement plus qu'un de chaque coté du plan sagittal (ou d'un plan parasagittal). Néanmoins, ne serait-ce que parce qu'il est possible de souhaiter traiter plusieurs étages vertébraux en même temps, cette expression ne devra pas être interprétée comme ne désignant que ces deux seules alternatives.

Les figures 5 à 14 montrent des exemples illustratifs et non limitatifs de diverses configurations possibles du système, c'est-à-dire de combinaisons d'implants utilisables. Les implants particuliers représentés dans chacune de ces combinaisons ne doivent pas être interprétés comme étant limitatifs, comme détaillé dans la présente demande et ce sont bien les combinaisons d'implants qui sont détaillées ci-après: La figure 5 (A à D)montre un exemple de configuration comprenant deux implants facettaires (IF) et un implant intersomatique (IS). L'implant intersomatique (IS) est un implant de type transforaminal sur les figures 5 (B à D). Les figures 7 (A à D), 9 (A à D) et 10 (A à E) montrent des exemples de configurations comprenant deux implants facettaires (IF) et deux implants intersomatiques (IS). Les implants intersomatiques (IS) sont des implants de type postérieur, à positionnement sensiblement parallèle, sur les figures 9 (B à D) et 10 (B à D), et de type postérieur à positionnement oblique ou de type transforaminal sur les figures 7 (B à E). Les figures 8 (A à E) montrent des exemples de configurations comprenant deux implants intersomatiques (IS) et un implant interépineux (IE). Les implants intersomatiques (IS) sont des implants de type postérieur, à positionnement sensiblement parallèle, sur ces figures mais il est possible de prévoir à la place un type postérieur à positionnement oblique ou un type transforaminal (non représentés).Les figures 11 (A à E) montrent des exemples de configurations comprenant deux implants facettaires (IF), un implant interépineux (IE) et deux implants intersomatiques (IS). L'implant intersomatique (IS) est un implant postérieur (comme par exemple celui des figures 8, 9 et 10), de même qu'il est possible de le remplacer par un implant intersomatique (IS) de type postérieur (à positionnement parallèle ou oblique). Cette avant-dernière possibilité est d'ailleurs d'autant plus réaliste qu'en ayant implanté deux implants intersomatiques, l'implantation d'un deuxième implant facettaire ne nécessiterait généralement pas d'incision supplémentaire. En revanche, selon les ablations ou résections nécessaires (ce qui dépend de la configuration particulière du rachis du patient), il n'est parfois pas possible de conserver les facettes des deux côtés et l'on peut donc être amené à n'utiliser qu'un seul implant facettaire, même si l'on a utilisé deux implants intersomatiques serait déjà réalisée postérieur ne rajouterait rallonger serait pas. Les figures 6 (A à D) montrent des exemples d'un tel système avec un seul implant facettaire, combiné dans ce cas à un seul implant intersomatique, qui est de type transforaminal.

Les figures 13A à 13D montrent des exemples de configuration comprenant deux implants intersomatiques et deux implants facettaires. Les figures 14A à 14E montrent des exemples de configuration comprenant deux implants intersomatiques et un implant interépineux. Dans ces exemples, l'implant intersomatique est de type postérieur et comprend un dispositif d'ancrage de type vis ; a contrario du dispositif d'ancrage des implants intersomatiques des autres figures qui est de type ancre, à l'exception des figures 2A à 2D. On notera que ce type de configuration est particulièrement avantageux car il atteint une majorité des buts visés par l'invention. En effet, cette configuration permet une stabilisation vertébrale efficace et rapide puisque ladite configuration permet d'augmenter la zone de l'espace discal stabilisée par deux implants peu invasifs, tout en permettant une implantation facile et rapide par le chirurgien. En effet, ce type de configuration permet de minimiser l'invasivité de l'intervention chirurgicale puisque le chirurgien peut se limiter à une seule incision, généralement médiane, le plus souvent dans le plan sagittal au niveau des épineuses, pour pouvoir implanter les deux implants intersomatiques postérieurs par les deux voies d'approches postérieures situées de part et d'autre des épineuses. Le chirurgien pourra bien entendu pratiquer une résection partielle des structures osseuses pour passer les implants intersomatiques, dont les dimensions pourront donc varier, notamment dans la gamme valeur détaillée dans la présente demande. Avantageusement, le chirurgien pourra passer par la même incision pour implanter ensuite au moins un implant facettaire et/ou au moins un implant interépineux (sur au moins un étage vertébral). Ainsi, cette configuration permet d'implanter efficacement et rapidement deux à cinq implants à travers une seule incision et ainsi obtenir une stabilisation vertébrale inégalable en si peu de temps. Ainsi, l'invention peut concerner également une méthode pour l'arthrodèse d'au moins deux vertèbres (VI, VS) adjacentes, comprenant typiquement une incision médiane au niveau d'au moins une épine dorsale (El, ES), parfois suivie d'une résection partielle des articualires ou des lames basales ou d'autres structures osseuses pour l'insertion de deux implants intersomatiques postérieurs. On notera qu'au cours de ce type d'intervention, le chirurgien peut préférer pratiquer une résection partielle des épineuses pour faciliter l'insertion des implants, en supprimant le ligament sus-épineux et une partie de l'extrémité libre de l'épineuse. Dans ce cas, on comprend qu'il n'est plus nécessaire d'utiliser un implant interépineux qui soit apte à être inséré depuis une seule face latérale des épineuses (ce qui vise à préserver le ligament sus-épineux et les épines) puisqu'il devient plus aisé de poser l'implant interépineux par translation parallèlement au plan sagittal. Ainsi, l'invention peut donc concerner l'utilisation d'une combinaison d'au moins un implant intersomatique postérieur avec au moins un implant interépineux comprenant des ailes fixes (non déployables). En effet, l'implant interépineux n'est alors pas limité aux types d'implants interépineux décrits en détail dans la présente demande, mais le système peut également comprendre tous types d'implants interépineux avec des éléments mobiles ou fixes de l'art antérieur (dont certains types d'implants interépineux avec des éléments mobile ou fixes de l'art antérieur sont décrit ci-dessous), comme par exemple les implants de l'art antérieur US5876404 et US2005203512. Ainsi, dans certains cas de résection partielle des épineuses, on peut implanter aussi des implants moins avantageux que la plupart des implants interépineux décrits dans la présente demande. En revanche, comme l'invention vise à une bonne stabilisation du rachis en vue de l'arthrodèse, on préférera que l'implant intersomatique comporte des moyens d'immobilisation des épineuses, par exemple tels que des moyens de crochetage ou des moyens de compression des épineuses ou encore des moyens de fixation ancrés dans les épineuses, dont des exemples illustratifs et non-limitatifs sont décrits dans la présente demande. La méthode comporte donc, suite à l'étape d'insertion d'au moins un implant intersomatique, une étape d'ancrage de cet implant via l'abord postérieur utilisé pour son implantation. Ces étapes peuvent être répétées de l'autre coté de pour une insertion d'un deuxième implant intersomatique par la même voie postérieure et un ancrage de cet implant. Néanmoins, il est possible de n'utiliser qu'un seul implant intersomatique postérieur, notamment si la méthode prévoit de l'orienter obliquement dans l'espace discal ou si cet implant est de type expansible dans le plan de l'espace discal, afin d'augmenter la zone stabilisée par l'implant. En effet, il est connu des implants intersomatiques postérieurs qui sont expansibles et on prévoira de préférence un ancrage de ces derniers pour l'utilisation dans un système selon la présente invention. Ensuite, la méthode peut comporter enfin l'introduction d'au moins un implant facettaire (interfacettaire ou transfacettaire) et/ou l'introduction de l'implant interépineux.

D'une manière générale, une particularité du système d'arthrodèse selon divers modes de réalisation est que l'on préfère en général qu'au moins deux des implants utilisés soient ancrés dans les structures osseuses. En particulier, on préfère un implant intersomatique ancré, c'est-à-dire fixé de façon solide, de préférence dans les deux plateaux vertébraux (un plateau de chacune des vertèbres adjacentes). De même, les implants transfacettaires forment un ancrage osseux et divers implants interfacettaires possèdent aussi un ancrage osseux ou une stabilisation (et ils permettent en général à terme une fusion des articulaires). Enfin, dans le cas d'un implant interépineux, on préfère qu'il comporte des moyens d'immobilisation des épineuses, de sorte à empêcher les mouvements de torsions et/ou de flexion du patient, en plus de l'extension qui est naturellement limitée par la présence de l'implant entre les épineuses si les dimensions sont correctement déterminées. Ainsi, de façon générale on préférera utiliser un implant intersomatique qui immobilise les corps vertébraux avec un implant facettaire et/ou un implant interépineux qui permettant également d'immobiliser la structure vertébrale dans laquelle ils sont implantés, de sorte à obtenir une immobilisation en plusieurs points et atteindre la plupart des buts que la présente invention vise à résoudre. C'est en particulier avec ce type d'immobilisation ou fixation synergique de plusieurs implants rachidiens que divers modes de réalisation de l'invention permettent de limiter les risques pour le patient tout en répondant à un maximum d'objectifs, notamment en termes de temps d'intervention chirurgicale, d'invasivité et de stabilité et/ou fiabilité.

Enfin, on notera que dans les exemples des figures 10 (A à E), les implants facettaires utilisés sont des implants de type transfacettaires, c'est-à-dire qu'ils sont implantés au travers des facettes des deux vertèbres adjacentes, alors que sur les autres figures les facettaires utilisés sont des implants de type interfacettaires, c'est-à-dire qu'ils sont implantés entre les facettes des deux vertèbres adjacentes. Bien entendu, des implants transfacettaires ou interfacettaires peuvent généralement être utilisés dans n'importe laquelle des combinaisons détaillées ci-dessus. On peut d'ailleurs aussi utiliser un implant interfacettaire d'un côté avec un implant transfacettaire de l'autre côté, tandis que les implants somatiques seront généralement les mêmes lorsqu'ils sont deux.

Selon un mode de réalisation préféré de la présente invention, ledit système est destiné pour l'arthrodèse d'au moins deux vertèbres (VI, VS) lombaires et privilégiant une implantation desdits implants pour la fusion osseuse par un abord mini-invasif, de préférence un abord transforaminal ou un abord postérieur. Plus spécifiquement, ledit système d'arthrodèse est destiné pour une fusion intervertébrale lombaire postérieure (en anglais, Posterior Lombar Interbody Fusion PLIF) ou une fusion intervertébrale lombaire transforaminale (en anglais, Tranforaminal Lombar Interbody Fusion TLIF).

Les divers implants utilisés dans un système selon divers modes de réalisation de l'invention pourront être différents de ceux représentés sur les figures de la présente demande et prendre des formes très variées, notamment telles que celles des nombreux implants intersomatiques (IS) interépineux (IE) et facettaires(IF) connus de l'art antérieur.

### Les différents types d'implants du système d'arthrodèse:

On parle ici de plusieurs implants et de plusieurs types d'implants car la présente demande décrit un système comprenant une combinaison de plusieurs types d'implants et peut, pour chaque type d'implant, utiliser plusieurs implants. Par exemple, on peut utiliser deux implants facettaires en combinaison avec deux implants intersomatiques et obtenir ainsi un système comprenant quatre implants, mais seulement deux types d'implants. De plus, on notera que l'on peut utiliser par exemple plusieurs types (au moins deux) d'implants et plusieurs implants de chaque type, mais que les implants d'un type donné (intersomatique, interépineux ou facettaire) peuvent ne pas être identiques entre eux. Ainsi, la présente demande prévoit une utilisation des implants détaillés plus loin en combinaison les uns avec les autres, mais également en combinaison avec d'autres implants (par exemple connus de l'art antérieur), mais de préférence des implants présentant les mêmes avantages de stabilité et/ou d'invasivité que ceux détaillés dans la présente description.

Dans certains modes de réalisation, le système comporte des implants tels que ceux représentés sur les figures de la présente demande, de façon à optimiser les effets synergiques entre les implants et fournir les avantages décrits en détail dans la présente demande. D'une manière générale, dans ces modes de réalisation préférés, le système d'arthrodèse rachidienne comprend donc plus spécifiquement, d'une part, au moins un implant de type intersomatique (IS) par exemple du type de ceux représentés de manière non-limitative sur les figures 1 (A, B, C, E et F) et 2 (A, B, C et D), et d'autre part, au moins un implant de type interépineux (IE), par exemple du type de ceux représentés non-limitativement sur les figures 3 (A, B, C et E), et/ou au moins un implant de type facettaire (IF) par exemple tel que ceux représentés non-limitativement sur les figures 4 (A, B, D et E) et 12 (A à D).

Divers modes de réalisation vont maintenant être décrits en référence aux figures de la présente demande, pour illustrer plus en détail les caractéristiques préférées pour les divers types d'implants.

Les dispositifs d'ancrage (1) (ou « système d'ancrage » ou « dispositifs de fixation » ou « vis » ou encore « ancres ») des implants intersomatiques (IS) peuvent être utilisés en combinaison les uns avec les autres ou en combinaison avec d'autres types de dispositifs de fixation et/ou d'implants intersomatiques, de la présente demande ou de l'art antérieur. En effet, il est possible d'utiliser par exemple ceux détaillés dans les figures et la description des demandes suivantes: EP2688521 (figures 1 et 5, page 7 - ligne 9 à page 8 - ligne 5 et page 8 - lignes 9 à 11), WO2010121028 (figures 1 et 9, paragraphes [74] à [76] et [104]), WO2002013732 (figures 1 à 4, page 4 - lignes 7 à 20), WO2013141990 (figures 3, 4 et 7, page 7 - ligne 8 à page 8 - ligne 10), WO2012094647 (figures 1 et 2, paragraphe [23]), WO2013072582 (figures 3, 4 et 11, page 10 - ligne 11 à page 11 - ligne 1), WO2011153536 (figures 1, 2 et 4, paragraphes [27] et [35]), WO2010090801 (figures 1, 2, 7 et 8, paragraphe [54]), FR2795627 (Figures 1 à 4, page 3 - ligne 28 à page 29 - ligne 11), US2014052260 (figure 1, paragraphe [32]), WO2013062716 (figures 1 à 8, paragraphes [5] et [34]), WO2011019411 (figure 1, paragraphe [33]), US2009265007 (figures 4 à 7, paragraphe [23]), CN102525624 (figures 1 à 7), US20150127107 (figures 1 à 3, paragraphe [38]), KR20140018668 (figures 1 et 2), WO2015164707 (figures 1, 3 et 5, paragraphes [8] et [55]), US5571109 (figure 1), US2005096745 (figure 1, paragraphe [3]), US2011282459 (figures 1 et 2 paragraphe [9]), US20120271422 (figures 1, 13 et 16), US20120215313 (figures 1 et 2, paragraphe [41]), US20070282449 (figures 1, 5 et 6) et US20020128712 (figures 1 et 4, paragraphe [36]). On notera que l'on préférera utiliser des dispositifs permettant de répondre au problème d'invasivité de manière similaire à ceux de la présente demande, c'est-à-dire grâce à une fixation réalisée par une approche dans le même plan que celui d'insertion de l'implant. Ainsi, il est prévu d'utiliser des systèmes d'ancrage comportant plusieurs dispositifs d'ancrage (1) qui peuvent être identiques ou différents, voire complémentaires entre eux. Les implants intersomatiques (IS) sont de préférence agencés pour recevoir un ou plusieurs de tels dispositifs ou systèmes d'ancrage (1), et incluent, de manière non exclusive, des cages intersomatiques (IS) configurées pour une implantation par voie postérieure ou transforaminale, comme décrit dans la présente demande, même s'il est parfois possible d'utiliser d'autres implants destinés à d'autres voies d'approches. Les implants intersomatiques (IS) postérieurs, de préférence ancrés, sont de préférence du type de ceux décrits dans la présente demande mais il est possible d'utiliser d'autres types d'implants interépineux, comme par exemple tels que détaillés dans les figures et la description des demandes suivantes: FR3016793, FR2954692, US2013245767 (figures 2 à 4 et 7, paragraphes [16], [18] et [42]), US20110040382 (figures 1 et 2, paragraphes [9], [80] et [105]), FR2866229 (figure 1) et US6045579 (figures 4 et 5). Les implants intersomatiques (IS) transforaminaux, de préférence ancrés, sont de préférence du type de ceux décrits dans la présente demande mais il est possible d'utiliser d'autres types d'implants interépineux, comme par exemple tels que détaillés dans les figures et la description des demandes suivantes: FR3016793, FR2954692, US2013018470 (figures 1 et 2, paragraphe [69]) et FR2866229 (figures 1 et 5).

Les implants interépineux (IE) sont de préférence du type de ceux décrits dans la présente demande mais il est possible d'utiliser d'autres types d'implants interépineux, comme par exemple tels que détaillés dans les figures et la description des demandes suivantes: US2009292316 (figures 1 et 2, paragraphes [10] et [51]), US5876404 (figures 21 et 22, page 1 - lignes 29 à 43 et page 7 - lignes 24 à 36), US2010106191 (figures 3 et 4 ; paragraphes [09] et [31], US2008114456 (figure 3, paragraphes [06] et [66]), US2005203512 (figures 1, 4 et 6, paragraphe [55]), WO2007109402 (figures 2 et 7, page 2 - lignes 1 à 12), WO200770819 (figures 4 et 17, page 2 -ligne 28 à page 3 - ligne 2), US2006271194 (figure 1, paragraphe [51]), US20110313458 (figures 1 et 9, paragraphes [11], [12] et [15]), US20090234389 (figures 2 et 3, paragraphes [5] et [13]), WO201230141 (figures 1 et 2) et KR20130112407 (figures 1 et 2). On notera que l'on utilisera de préférence des implants interépineux permettant de répondre au problème d'invasivité de manière similaire à ceux de la présente demande, c'est-à-dire grâce à une implantation depuis une seule face latérale des épines en limitant les lésions des ligaments inter-épineux et sus-épineux.

De même, les implants facettaires (IF) sont de préférence du type de ceux décrits dans la présente demande mais il est possible d'utiliser d'autres types d'implants facettaires car ce type d'implants nécessite en général simplement d'être placé entre les surfaces articulaires ou au travers de ces dernières, sans nécessiter une large voie d'approche aux surfaces articulaires. Ainsi, il est possible par exemple d'utiliser des implants interépineux du type de ceux détaillés dans les figures et la description des demandes suivantes US2012010662 (figures 1, 4 et 5, paragraphes [62] et [81]), EP2589351 (figures 6 et 19, paragraphes [3] et [93]), EP1718228 (figures 3 à 5, paragraphes [2] et [14]), US7291149 (figures 1 à 3 et 12, page - lignes 20 à 35 et page 4 - ligne 56 à page 5 - ligne 2), US2012010659 (figures 2 et 3, paragraphe [119] et [121]), US2011004247 (figure 2, paragraphe [5]), US2013116732 (figures 1 et 2, paragraphes [14], [15] et [37]), US2008255622 (figure 2, paragraphe [8]), US20120232599 (figure 1, paragraphes [5] et [52]), US2014025113 (figure 2, paragraphe [47]), CA2868610 (figure 1, page 12 - ligne 13 à page 13 - ligne 12), US2005234459 (figure 2, paragraphes [4] et [42])), et WO2012154653 (figures 2, 6 et 21, paragraphes [3] et [25]). Cependant, les implants décrits ici sont préférés pour leur solidité et/ou pour la stabilité et la fiabilité de la fusion articulaire.

Chacun de ces types d'implants (objets ou groupes d'objets) peut comporter divers modes de réalisation possibles, relatifs à un objet donné. Chacun des objets comporte divers éléments (généralement constitutifs de l'objet) caractérisés par au moins une caractéristique technique. Chaque objet (d'un groupe donné) concerné par au moins une caractéristique technique peut être associé à au moins un autre objet (du même ou d'un autre groupe), par exemple à l'égard d'au moins une caractéristique technique complémentaire, de telle sorte que les groupes d'objet partagent un concept inventif commun. La présente demande peut donc concerner un ensemble comprenant au moins deux de ces objets. Les divers éléments (par exemple, un corps, une plaque, une butée, une fente, un chanfrein ou biseau, etc.) ainsi que leurs caractéristiques techniques (par exemple, une courbure, une orientation, une longueur, une largeur, une hauteur, une forme, une dimension etc.) sont décrits avec plus de détails ci-après dans la présente demande. Au moins une caractéristique technique (ou combinaison de caractéristiques), correspondant par exemple à un élément d'un objet donné, résout généralement au moins un problème technique, en particulier parmi ceux mentionnés dans le préambule de la présente demande. La présente demande décrit donc divers modes de réalisation ou configurations pour chaque objet ou groupe d'objets, en spécifiant au moins une caractéristique technique d'au moins un élément. On comprendra à la lecture de la présente demande que chacune des caractéristiques techniques de chaque élément, décrite dans au moins un mode de réalisation ou une configuration, pourra être isolée des autres caractéristiques de l'objet concerné (ou des objets concernés et/ou associés) par ledit mode de réalisation ou ladite configuration (et concernant donc le même élément ou un élément différent) et/ou pourra être combinée avec n'importe quelle autre caractéristique technique décrite ici, dans divers modes de réalisation ou configurations, à moins que l'inverse ne soit explicitement mentionné, ou que ces caractéristiques ne soient incompatibles entre elles et/ou que leur combinaison ne fonctionne pas. En effet, les adaptations structurelles qui peuvent en particulier être requises par de tels isolements ou combinaisons de caractéristiques sont directement dérivables de l'appréciation des considérations fonctionnelles fournies dans la présente demande. De même, bien que certaines caractéristiques techniques soient discutées ici en référence au dispositif d'ancrage, elles pourront être incorporées dans divers modes de réalisation ou configuration des systèmes d'ancrage. D'une manière générale, la (ou les) caractéristique(s) technique(s) spécifique(s) concernant un élément donné ne doivent pas être considérées comme exclusives de celles concernant un autre élément, ni d'autres caractéristiques techniques concernant le même, sauf lorsqu'il apparaît clairement que la combinaison est impossible ou non-fonctionnelle. Bien que la présente demande détaille divers modes de réalisation ou configurations de l'invention (incluant des modes préférés), son esprit et sa portée ne doivent pas être limités aux exemples donnés.

### Les implants de type intersomatiques (IS) :

Divers modes de réalisation de l'implant intersomatiques (IS) selon la présente invention peuvent être utilisés pour l'arthrodèse d'au moins deux vertèbres (VI, VS) adjacentes, comme représenté de manière non-limitative dans les figures 1A à 1F et 2A à 2F.

Dans divers modes de réalisation, on préfère utiliser des implants intersomatiques munis d'ancrages osseux. Néanmoins, à cause de l'encombrement, l'ancrage sera de préférence choisi parmi des dispositifs d'ancrage sous la forme d'une ancre courbe car elle permet une implantation de l'ancre par un abord qui se fait sensiblement dans le plan de l'espace discal. Ainsi, on utilise par exemple des implants intersomatiques (IS), destinés à être implanté dans l'espace discal entre les vertèbres (VI, VS) adjacentes et comportant au moins une paroi périphérique, dont au moins une partie, dite postérieure, comporte au moins un passage (40) rectiligne de forme, dimensions et orientation complémentaires aux formes et dimensions d'au moins un dispositif d'ancrage ou ancre ou vis (1) comportant au moins un corps (10) rigide et allongé selon un axe longitudinal s'étendant entre une première extrémité, dite antérieure, et une seconde extrémité, dite postérieure, ledit corps (10) étant inséré sans déformation dans ledit passage (40), sensiblement dans le plan de l'implant intersomatique (IS), par coulissement depuis ladite partie postérieure de l'implant intersomatique (IS), ledit passage (40) traversant l'implant intersomatique (IS) depuis la périphérie vers une surface supérieure ou inférieure de sorte que l'extrémité antérieure de ledit corps (10) pénètre dans une desdites vertèbres (VI, VS) adjacentes, tandis que l'extrémité postérieure reste dans ledit passage (40) et retient ledit implant intersomatique (IS) contre ladite vertèbre (VI, VS).

Les termes « ancrer ou ancrage » se définissent dans la présente demande, comme une implantation solide et suffisamment profonde dans les plateaux vertébraux pour assurer un bon maintien de l'implant plaqué contre ces vertèbres, par opposition à de simples dents antidérapantes fréquemment utilisée que l'on retrouve d'ailleurs en générale sous la référence (42) de la présente demande. Un implant ancré, selon la définition de la présente demande, comporte typiquement au moins un dispositif d'ancrage, tel qu'une vis ou une ancre. Cette dernière étant de préférence et généralement formée par une plaque courbe et rigide. La rigidité de ce type d'ancre permet une fixation efficace, généralement plus efficace que les agrafes ou autres dispositifs fins et/ou relativement flexibles, voire fragiles. Ainsi, ce type de solution comporte un dispositif d'ancrage d'implant intervertébral dans les vertèbres, comportant un corps comprenant au moins une plaque courbe et allongée selon un axe longitudinal, le dispositif d'ancrage étant destiné à être inséré au travers d'un passage traversant au moins une portion de l'implant, de sorte que l'extrémité antérieure du dispositif d'ancrage pénètre le tissus osseux, alors que l'extrémité postérieure du dispositif d'ancrage reste dans l'implant pour le retenir.

Dans certains modes de réalisation, en alternative, l'implant intersomatique (IS) peut être fixé contre les vertèbres à l'aide d'au moins une vis d'ancrage vertébral connue de l'art antérieur, généralement utilisée avec un tournevis à cardan à cause de l'encombrement, même si l'on préfère en général utiliser au moins une ancre (1) tel que décrite ci-dessus et ci-dessous.

Ledit dispositif d'ancrage (1) est destiné à être ancré dans une des vertèbres de façon à fixer l'implant sur/contre cette vertèbre. Divers modes de réalisation de dispositifs d'ancrage (1) selon l'invention comportent au moins un corps (10) rigide et allongé, de préférence courbe et en forme de plaque ou clou (de section rectangulaire, circulaire, carrée, polygonale ou en T, en L, en U ou même en H), agencée pour pénétrer dans une vertèbre au travers d'un implant pour retenir cet implant contre cette vertèbre. Le dispositif d'ancrage (1) d'implant intersomatique (IS) intervertébral dans les vertèbres, est parfois désigné dans la présente demande sous le terme « ancre » (1) en référence à sa fonction d'ancrage, sans induire de limitation quelconque. Divers modes de réalisation d'ancres ont été décrits dans les demandes publiées WO2008/149223, WO2011/080535 et WO2013/124453 déposées par la demanderesse de la présente demande, mais également dans d'autres demandes non publiées la présente demande fournit des modes de réalisation qui améliorent les solutions fournies par ces demandes et s'appliquent plus facilement à tout d'implant, en particulier spinal (spinal étant utilisé ici comme identique à rachidien ou vertébral) mais éventuellement avec d'autres applications. Ainsi, la présente invention fournit des améliorations de diverses structures et méthodes qui peuvent être utilisées dans diverses applications pour réduire l'invasivité des opérations nécessaires à l'implantation de l'implant et de l'ancre et pour améliorer la fiabilité des solutions proposées.

Le terme « rigide » est donc ici utilisé pour préciser que l'on préfère que l'ancre (1) passe au travers de l'implant (IS) sans devoir subir de déformation élastique ou de quelconque déformation. De plus, on entend par là qu'elle puisse offrir une rigidité et une solidité suffisante pour résister aux contraintes qui seront exercées dessus, sans se déformer ou du moins sans se déformer de manière trop importante. Le passage (40) dans l'implant (IS) pourra alors, pour accueillir cette ancre (1), de préférence courbe, mais il pourra être rectiligne du moment que ses dimensions soient adaptées à celles de l'ancre et au(x) rayon(s) de courbure de cette dernière.

Dans divers modes de réalisation, l'ancre (1) comporte un corps comprenant au moins un corps (10), de préférence rigide, allongée selon un axe longitudinal. Cet axe longitudinal de l'ancre (1) s'étend entre une première extrémité, désignée ici comme « extrémité antérieure », destinée à pénétrer dans une vertèbre et une seconde extrémité, désignée ici comme « extrémité postérieure ». On notera que les désignations des extrémités « postérieure » et « antérieure » de l'ancre (1) et, de l'implant (IS) sont utilisées dans la présente demande en référence au sens selon lequel l'ancre (1) est insérée. Ainsi, pour l'ancre (1), la première extrémité, dite antérieure, est celle destinée à être insérée en premier et destinée à pénétrer dans une vertèbre pour fixer un implant. Concernant l'implant intersomatique, sa paroi ou son extrémité désignée comme postérieure est celle comportant une ouverture d'un passage (40) pour l'insertion de l'ancre (1), que cette paroi soit réellement postérieure à l'implant ou non lors de son déploiement. Dans le cas des cages intersomatiques (IS) ou prothèses de disque ou plaques décrites dans la présente demande, cette extrémité postérieure peut être effectivement disposée vers l'arrière du patient ou non, notamment pour les cages qui sont essentiellement destinées à une implantation par voie postérieure ou transforaminale.

Certains modes de réalisation d'implants intersomatique concernant une cage intersomatique qui sont agencé pour une insertion dans l'espace discal par voie transforaminale et l'extrémité postérieure sera par conséquent disposée sur un côté latéral et arrière des vertèbres alors que l'extrémité antérieure sera disposée à proximité du côté avant et latéral opposé. Néanmoins, on utilise ici quand même les termes « antérieur » et « postérieur » car ils sont plus faciles à comprendre d'un point de vue de l'implantation et peuvent être utilisés de façon pratique et communément à l'ancre (1) et à l'implant (IS), quelle que soit la voie d'implantation choisie. Par conséquent, les termes « antérieur » et « postérieur » ne sont pas destinés à référer simplement au patient ou à une de ses caractéristiques anatomiques, mais au sens d'insertion de l'ancre dans l'implant (que cet implant soit lui-même implanté selon un axe antéro-postérieur ou non). D'autre part, on désigne généralement ici par les termes « hauteur » et « épaisseur » les dimensions des éléments selon une orientation parallèle à l'axe du rachis (une fois implantés dedans) et les termes « supérieure » et « inférieure » (ou dessus et dessous) sont généralement définis également selon cette orientation (verticale lorsque le patient est debout), sans implication limitative pour l'invention. De même, les termes « vertical » et « horizontal » sont utilisés de manière non limitative en référence à l'axe du rachis en considérant le patient debout. D'autre part, on désigne par les termes « largeur » et « longueur » des dimensions selon un plan perpendiculaire à l'axe du rachis (un plan transversal), avec la largeur étant généralement dans la direction médio-latérale alors que la longueur sera dans la direction antéro-postérieure, sans que cette définition conventionnelle ait la moindre implication limitative pour l'invention. On notera également qu'il est fait référence ici à un axe longitudinal entre deux extrémités et que cet axe longitudinal correspond éventuellement à un axe antéro-postérieur de l'ancre (1), mais que cet axe est en fait oblique en général puisque l'ancre est souvent insérée depuis la périphérie du rachis dans une structure vertébrale (un corps vertébral le plus souvent et généralement dans un plateau vertébral). De plus, cet axe de l'ancre suit même un trajet courbe dans de nombreux modes de réalisation et il est donc désigné comme antéro-postérieur par rapport aux extrémités de l'ancre plutôt qu'en référence au rachis. De même, l'axe du passage est désigné en utilisant les mêmes références alors qu'il est oblique et qu'il peut être curviligne ou rectiligne. On notera aussi qu'on étend cette définition également à l'implant (IS) toujours en référence au sens d'insertion de l'ancre (1). On notera également que le terme « sensiblement » ou « substantiellement » est régulièrement utilisé dans la présente description, notamment concernant une caractéristique telle qu'une orientation ou une direction, de manière à indiquer que la caractéristique concernée peut en fait être légèrement différente et ne pas être exactement comme désigné (par exemple, l'expression « sensiblement perpendiculaire » doit être interprétée comme « au moins approximativement perpendiculaire » car il peut être possible de choisir une orientation qui ne soit pas exactement perpendiculaire pour pouvoir néanmoins remplir sensiblement la même fonction). De plus, les termes tels que le terme "sensiblement" utilisés dans la présente demande peuvent également être interprétés comme définissant que la caractéristiques technique peut être « en général » (« généralement »), et souvent « de préférence », comme indiqué, mais que d'autres modes de réalisation ou configurations peuvent être dans la portée de la présente invention.

Dans certains modes de réalisation, ledit corps (10) du dispositif d'ancrage (1) comporte au moins une nervure (16) ou second corps coopérant avec au moins une rainure ménagée dans le passage (40) de l'implant (2). Ladite nervure (16) est de préférence destinée au moins à limiter (voire empêcher) le déplacement transversal de l'ancre (1) (et donc également de l'implant) par rapport à la vertèbre. La nervure peut également être configurée et déployée pour rigidifier l'ancre (1). Par exemple, les figures 1D et 2D montrent des dispositifs (1) d'ancrage munis d'au moins une nervure (16). La figure 1E montre un implant dont le passage (40) possède deux rainures pour le passage de telles rainures du dispositif d'ancrage. Une telle nervure peut en fait être sur diverses portions de l'ancre (1) et avoir diverses dimensions pour fournir une bonne stabilité de l'ancre (1) dans les vertèbres. Ainsi, cette nervure peut en fait former un second corps (16) longeant le premier corps (10) du dispositif d'ancrage (1). Ainsi, dans certains modes de réalisation, l'ancre (1) comporte au moins deux corps (10, 16) dont les axes longitudinaux sont parallèles entre eux, mais dont les axes transversaux ne sont pas parallèles entre eux. Par exemple, les figures 1B, 1D, et 2E montrent des ancres (1) comportant deux corps (10, 16) perpendiculaires entre elles. De préférence, les axes transversaux des deux corps (10, 16) sont perpendiculaires entre eux, conférant une section en L au dispositif d'ancrage (1), mais ils peuvent également présenter un angle différent de 90°, par exemple en conférant au dispositif une section en V. De même, il est envisageable qu'il s'agisse en fait d'un seul et même corps mais qui est courbé dans cette dimension transversale, de sorte que le dispositif possède une section en C (et il est clair qu'il est possible de prévoir d'autres formes de section, en H, en U, etc.). Ce type d'agencement peut être utile car les corps rigides utilisés dans la présente invention sont plus stables que d'autres moyens de fixation moins solides, tels que des clous ou des agrafes, mais, surtout, le fait d'avoir une ancre dont la largeur (la dimension transversale à son axe longitudinal) présente 2 bords d'orientations différentes (par le fait qu'elle comporte deux corps non parallèles ou un corps courbé ou une nervure), permet de s'opposer aux mouvements de l'ancre dans l'os selon au moins deux directions différentes. Ainsi, l'ancre est nettement stabilisée dans l'os et ne risque pas d'entailler ou découper les vertèbres par des mouvements latéraux. On désigne donc ici par le terme de « deux corps » et de « section en L » cette possibilité de fournir une seconde surface s'opposant aux mouvements selon une seconde direction, qu'il s'agisse en fait d'un seul corps courbe ou bien de deux corps (d'orientations non parallèles mais variables, par exemple en L ou en V). Ainsi, certains modes de réalisation de l'invention concerne un dispositif d'ancrage (1) dont le corps comporte un deuxième corps (16) allongé selon ledit axe longitudinal du premier corps (10) et s'étendant entre l'extrémité antérieure et l'extrémité postérieure, le deuxième corps (16) étant solidaire du premier corps (10) et non parallèle à au premier corps (10), conférant au dispositif une section en forme de L, de V ou de C, complémentaire de la section interne du passage dans l'implant (IS). Ce type d'agencement avantageux peut être envisagé quel que soit le type de butées utilisés, c'est-à-dire comprenant ou non une butée (13) décrite dans la présente demande en coopération avec un dispositif (ou moyen) de verrouillage (4).

D'une autre manière, le dispositif d'ancrage (1) selon divers modes de réalisation de l'invention comporte au moins une butée de retenu (13) (parfois limitée à une simple surface) agencé pour verrouiller l'ancre (1) par rapport à l'implant (ou inversement).Le corps (10) est configuré, dans certains modes de réalisation, pour que son extrémité antérieure pénètre dans au moins une vertèbre alors que son extrémité postérieure reste dans le passage (40) de l'implant (IS) ou contre un bord de l'implant (IS), en pressant ainsi ledit implant (IS) contre ladite vertèbre grâce à au moins une butée de retenue (13), orientée non parallèlement à (ou en formant angle avec) l'axe longitudinal (L) du corps (10) et appuyant contre une surface complémentaire de l'implant (IS) (e.g., sur un bord ou dans le passage (40) de l'implant).De préférence sur l'extrémité postérieure du corps (10) du dispositif d'ancrage (1) comporte au moins une butée de retrait (13) configurée pour retenir ou verrouiller l'ancre (1) dans l'implant (IS) en pressant l'implant intersomatique (IS) contre les vertèbres (VI,VS), comme représenté dans les figures 1A, 2E et 2F. Une telle retenue ou un tel verrouillage de l'ancre (1) dans l'implant peut être obtenu dans divers modes de réalisation par différents types de loquet, verrou, butée, etc. Dans certains modes de réalisation avantageux, cette retenue ou ce verrouillage est obtenu par au moins une butée de retrait (13) qui peut être orientée selon un angle par rapport (i.e., non parallèlement) à l'axe longitudinal (L) du corps (10) et configuré pour coopérer avec une surface complémentaire de l'implant et retenir le dispositif (1) dans l'implant.

Dans certains modes de réalisation de l'invention, le corps (10) du dispositif d'ancrage (1) comporte au moins une butée de retrait (12) flexible(s) orientée(s) formant une butée s'opposant au retrait du dispositif d'ancrage(1). Comme particulièrement visible sur les figures 2E et 2F, cette butée(12) flexible pourra être présente sur les deux côtés latéraux du corps(10).

Suivant un mode de réalisation représentée sur les figures 1C et 1D, l'implant intersomatique comporte un renfort (41) traversant sa cavité de part en part et qui peut être agencé pour renforcer la paroi périphérique de l'implant. Ce renfort (41) pourra avoir différentes formes et orientations et pourra par exemple être orientée selon l'axe d'insertion de l'implant (IS) entre les vertèbres. Dans divers modes de réalisation, le renfort (41) peut avoir hauteur moins importante que le reste de l'implant (IS). Cette hauteur moins importante du renfort (41) par rapport au reste de l'implant permet à l'implant d'épouser d'éventuelles irrégularités de formes des plateaux vertébraux.

Selon une particularité de l'invention, l'implant intersomatique (IS) peut avoir différentes formes tant qu'elles possèdent un passage (40) comporte au moins un moyen d'accrochage (44) destiné à coopérer avec une extrémité de préhension d'une instrumentation d'implantation. Ces moyens d'accrochage (44) pourront, selon les modes de réalisation, être associés à une forme particulière de l'implant (IS) ou du corps (10) à proximité de ces moyens d'accrochage (44) pour permettre une bonne coopération avec l'instrumentation ou même comporter une de ces formes particulières coopérant avec des formes complémentaires de l'instrumentation. De même, comme mentionné précédemment, l'implant intersomatique (IS) pourra comporter une cavité en son centre ou non, dans la mesure où il est fréquent d'implanter plusieurs implants intersomatiques dans un même espace intervertébral (pour autant que les dimensions le permettent). Les cages ainsi implantées sont généralement utilisées pour refermer du tissu osseux (greffon) qui va croître à l'intérieur de l'espace intervertébral et permettre une fusion (arthrodèse) des deux vertèbres entre lesquelles il est implanté. Le but de l'implant (IS) est de restaurer ou maintenir un espace entre les vertèbres.

Dans un mode de réalisation de l'invention, le dispositif d'ancrage(1) comporte au moins une butée (14) (parfois limitée à une simple surface) complémentaire d'une butée non visible sur les figures (ou surface également) d'un moyen (ou dispositif) de verrouillage (4) agencé pour verrouiller l'ancre (1) par rapport à l'implant (ou inversement). Ce moyen de verrouillage (4) est situé sur ou dans l'implant intersomatique lui-même, c'est-à-dire que le moyen de verrouillage (4) est logé dans un logement à l'intérieur de l'implant intersomatique (IS). Ce moyen de verrouillage (4) comprend de préférence un corps retenu dans l'implant et muni d'au moins une portion flexible et d'au moins une butée (49), coopérant avec ladite butée (14) du dispositif (1), en général grâce au contact de leurs surfaces de butée complémentaires (49) pour verrouiller le dispositif (1) par rapport à l'implant (IS). Dans divers modes de réalisation, il est tiré avantage de cette flexibilité qui permet que le moyen de verrouillage facilite le passage de l'ancre avant que sa butée ne s'engage avec la butée complémentaire de l'ancre. Par exemple, l'insertion du dispositif d'ancrage (1) dans le passage (dans lequel le moyen de verrouillage dépasse au moins légèrement) permet de repousser ladite butée (49) du moyen de verrouillage (4) et permet également l'engagement réciproque des deux butées (14, 49), de l'ancre et du moyen de verrouillage (4), lorsqu'elles se retrouvent l'une en face de l'autre, par le retour élastique de la portion flexible. Dans un autre exemple, comme détaillé plus loin, il est possible de pousser le moyen de verrouillage par d'autres moyens que le corps de l'ancre elle-même, comme par exemple à l'aide d'un outil, et lorsque l'action exercée sur le moyen de verrouillage est relâchée, celui-ci verrouille l'ancre amenée en position finale dans l'implant. Ainsi extrémité postérieure dudit corps (10) du dispositif d'ancrage (1) comporte au moins une butée (14) orientée non parallèlement à l'axe longitudinal dudit corps (10) et complémentaire d'au moins une butée (49) d'au moins un moyen de verrouillage (4) du dispositif (1) par rapport à l'implant intersomatique (IS), ledit moyen de verrouillage (4) qui équipe l'implant (IS) étant muni d'au moins une portion flexible permettant, d'une part, de repousser ladite butée du moyen de verrouillage (4) pour l'insertion du dispositif (1) d'ancrage dans le passage (40), et d'autre part, l'engagement réciproque des deux butées (14, 49) lorsqu'elles se retrouvent l'une en face de l'autre, par le retour élastique de la portion flexible.

Avant la croissance du greffon et la fusion des vertèbres, la cage (1A, 1B) doit rester bien en place dans l'espace discal et divers modes de réalisation de la présente invention facilitent son immobilisation. Avant l'implantation du dispositif d'ancrage (1) permettant de maintenir l'implant (IS) en position, il existe parfois un risque que l'implant ne bouge dans l'espace discal. Dans certains modes de réalisation, au moins une des surfaces supérieure et inférieure de la paroi de l'implant (IS) comportera des crans ou dents (42) évitant le déplacement de l'implant entre les vertèbres entre lesquelles elle est destinée à être implantée. Ainsi, au moins une des surfaces supérieure et inférieure de la paroi périphérique comporte des crans (42) évitant le déplacement de l'implant intersomatique (IS) entre les vertèbres entre lesquelles elle est destinée à être implantée. Selon différents modes de réalisation, ces crans (42) ou autres moyens de stabilisation pourront avoir différentes orientations, de façon à éviter le déplacement de l'implant (IS) dans une ou plusieurs directions. Par exemple, les crans (42) pourront être sensiblement parallèles entre eux et orientés tous perpendiculairement à l'axe d'insertion de l'implant (IS), mais les crans (42) pourront au contraire avoir des orientations différentes sur différentes portions de l'implant (IS), de façon à éviter le déplacement dans n'importe quelle direction. On notera que sur les diverses figures de la présente demande, les exemples de cages représentées comportent des crans sur la totalité ou quasi-totalité de leurs surfaces de contact vertébral, mais pas sur la paroi périphérique de la cage. La partie postérieure des surfaces de contact vertébral de la cage ne comporte pas de cran sur ces exemples. Cependant, il est possible dans divers modes de réalisation de ménager des crans sur cette partie ou d'autres parties périphériques, pourvu que ces crans n'interfèrent pas avec les diverses butées, nervures, et/ou autres éléments et caractéristiques qui peuvent être configurés sur ces implants et/ou sur les ancres qui peuvent leur être associées.

Dans certaines situations, notamment en fonction des vertèbres entre lesquelles l'implant (IS) doit être implanté, il est souhaitable que l'implant impose, épouse ou corrige une lordose, une cyphose ou même une scoliose, en plus de maintenir l'espace entre les vertèbres. Ainsi, selon les vertèbres entre lesquelles l'implant intersomatique (IS) doit être implanté, il est souhaitable que l'implant permette d'imposer une lordose ou une cyphose en plus de maintenir l'espace entre les vertèbres. Certains modes de réalisation les plans moyens passant par les surfaces supérieure et inférieure de l'implant (IS) forment un angle (A1) orienté selon un axe antéropostérieur de l'implant intersomatique (IS), permettant d'imposer une lordose ou une cyphose aux vertèbres entre lesquelles l'implant intersomatique (IS) est destiné à être implantée. Selon un mode de réalisation de l'invention, les plans moyens passant par les surfaces supérieure et inférieure de l'implant intersomatique (IS) sont sensiblement parallèles entre eux.

Dans certains modes de réalisation, la paroi périphérique comporte deux passages (40) orientés chacun vers une des surfaces supérieure et inférieure, de façon à permettre l'ancrage du dispositif d'ancrage (1) dans chacune des vertèbres entre lesquelles l'implant intersomatique (IS) est destinée à être implanté. Dans d'autres modes de réalisation, la paroi périphérique comporte au moins deux passages (40), situés à côté l'une et l'autre, chacun d'entre eux définissant un axe d'insertion possible du dispositif d'ancrage (1) dans l'implant intersomatique (IS) et, indirectement, un axe possible d'insertion de l'implant intersomatique (IS) entre les vertèbres.

De plus, dans certains modes de réalisation, la paroi périphérique comporte au moins un chanfrein (46) sur au moins une portion périphérique d'au moins une de ses surfaces supérieure et inférieure, de façon à faciliter l'insertion de l'implant intersomatique (IS) entre les vertèbres. Comme particulièrement visible sur les figures1B et 2B, ce chanfrein (46) de l'implant pourra être situé sensiblement dans l'axe d'implantation de l'implant. Ce chanfrein (46) ou profil biseauté permet de faciliter l'implantation de l'implant intersomatique en lui conférant une hauteur sensiblement plus faible sur son bord d'attaque (celui destiné à être inséré en premier) que sur le reste de l'implant. Le corps de l'implant intersomatique (IS) comporte, au niveau d'une partie antérieure (selon la convention de directions décrites ailleurs dans la présente demande, donc opposée à la partie postérieure comportant le passage (40) pour l'ancre (1)), au moins une portion biseautée (46), comme par exemple au moins un chanfrein (46) sur au moins une portion périphérique d'au moins une de ses surfaces supérieure et inférieure, de façon à faciliter l'insertion de l'implant (IS) entre les vertèbres (VI, VS). On notera que la portion biseautée (46) sur au moins une des surfaces supérieure et inférieure ne doit pas être trop grande par rapport aux dimensions du corps (par exemple une longueur inférieure à 1/3 de la longueur de l'implant) pour laisser une surface de contact suffisante des surfaces supérieure et inférieure avec les plateaux vertébraux. On a dans un autre mode de réalisation de la présente invention, dans lequel seulement une portion de la jonction entre, d'une part, au moins une des surfaces supérieure et inférieure et, d'autre part, la partie antérieure de la cage, qui est biseautée (par exemple le tiers antérieur dans le cas d'une cage intersomatique).

Comme particulièrement visible sur l'exemple de la cage intersomatique (IS) des figures 5B et 5D, l'extrémité antérieure de la cage a sensiblement la forme d'un biseau ou un chanfrein ou une pointe d'obus (46), pour optimiser la pénétration de la cage entre les vertèbres (VI, VS), notamment lorsque l'écartement desdites vertèbres est insuffisant. Le chanfrein ou biseau (46) peut en fait être présent sur les deux surfaces inférieure et supérieure de l'implant (IS). Ce chanfrein (46) ou profil biseauté permet de faciliter l'implantation de l'implant (IS) en lui conférant une hauteur sensiblement plus faible sur son bord d'attaque (celui destiné à être inséré en premier) que sur le reste de la cage. De plus, on peut également biseauter les faces latérales au niveau de l'extrémité antérieure de l'implant pour qu'il ait une forme d'obus facilitant sa pénétration entre les vertèbres. D'autre part, il est possible de biseauter au moins une partie des jonctions d'au moins certaines des faces latérales avec les surfaces supérieure et inférieure. En particulier, on souhaite parfois insérer l'implant dans une orientation pivotée à 90° autour de son axe longitudinal par rapport à la position finale (celle dans laquelle les surfaces supérieure et inférieure sont au contact des vertèbres adjacentes). En effet, comme expliqué précédemment, les dimensions de la cage destinée à une implantation par voie postérieure ou transforaminale peuvent être telles que les dimensions de la hauteur de la cage sont supérieures à celle de la largeur de la cage. Il peut donc être souhaitable d'insérer d'abord la cage avec ses faces latérales disposées vers le haut et le bas du rachis (les faces supérieure et inférieure se retrouvant disposées latéralement par rapport au rachis), et pivoter ensuite la cage pour restaurer la hauteur de l'espace intervertébral à la valeur souhaitée (obtenue grâce au fait que la hauteur de la cage a la valeur choisie). On insère donc l'implant dans une orientation pivotée à 90° autour de son axe longitudinal par rapport à la position finale, puis on la pivote pour la placer dans sa position finale une fois dans l'espace discal. Dans ce type d'implantation, il peut être souhaitable qu'au moins une portion d'au moins une partie des jonctions entre les faces latérales et les surfaces supérieure et inférieure soit biseautée pour faciliter la rotation de l'implant entre les vertèbres. On peut prévoir de tels biseaux ou des formes arrondies ou toute forme pour la cage, même si ce n'est pas ce type d'implantation qui est prévu, mais on préfère en général une cage qui offre le maximum de surface de contact pour une taille donnée et on préfère donc des jonctions qui ne sont pas trop arrondis. Il est alors préférable de prévoir de tels biseaux lorsqu'on prévoit cette rotation au cours de l'implantation lors d'une insertion de l'implant (IS) dans une position pivotée à 90° autour de son axe longitudinal par rapport à la position finale dans laquelle les surfaces supérieure et inférieure sont au contact des vertèbres adjacentes entre lesquelles l'implant (IS) est destiné à être implanté. En général, il suffit que seulement une partie des jonctions soit biseauté, comme par exemple une seule jonction sur les deux jonctions entre les faces latérales et la surface supérieure et une seule jonction sur les deux jonctions entre les faces latérales et la surface inférieure. On choisit alors de préférence les jonctions qui sont opposées (la jonction face gauche/surface inférieure opposée à la jonction face droite/face supérieure, par exemple), comme par exemple visible sur la figure 1B. De plus, il suffit, en général et notamment lorsque les surfaces supérieure et inférieure sont inclinées l'une par rapport à l'autre (e.g., lorsque l'implant est moins épais à sa extrémité postérieure qu'à son extrémité antérieure), que seule une portion de ces jonctions soit biseautée. En effet, il suffit de biseauter la portion au niveau de laquelle la cage est la plus épaisse, comme par exemple visible sur la figure 1B.

D'autre part, dans certains modes de réalisation, le corps de l'ancre (1) est muni de crans ou dents (11) orientés de façon à s'opposer au retrait de l'ancre (1) une fois implantée dans une vertèbre, visible sur les figures 2E et 2F.

On notera d'une manière générale que les passages, les trous, les crans, les dents, les butées, les logements, les pattes et autres éléments des divers objets de l'invention (ancres, implants, instruments...) pourront être formés par diverses méthodes comme par usinage, perçage, moulage, soudure, etc. et que les exemples donnés ici ne doivent pas être interprétés de façon limitative.

### Les implants de type facettaire (IF) :

Divers modes de réalisation de l'implant Facettaire (IF) selon la présente invention peuvent être utilisés pour l'arthrodèse d'au moins deux vertèbres (VI, VS) adjacentes, comme représenté de manière non-limitative sur les figures 4A à 4E et 12A à 12D.

On notera que l'on peut prévoir un implant inter-facettaire et/ou un implant transfacettaire. En effet, La présente description détaille notamment des implants, dits « facettaires », destinés à être implantés entre les facettes articulaires des vertèbres (implants dits « inter-facettaires ») et/ou implantés au travers de ces facettes articulaires des vertèbres (implants dits « transfacettaires »). Divers modes de réalisation de la présente demande sont également adaptés à une implantation dans les pédicules vertébraux (implants dits « pédiculaires ») ou au niveau de l'articulation sacro-iliaque ou dans divers types de structures osseuses, rachidiennes ou non, bien que les caractéristiques des implants décrits dans la présente demande les rendent particulièrement utiles pour leur utilisation dans le rachis.

Par exemple, il est connu de l'art antérieur, comme par exemple du brevet FR2726171B1, des implants sous forme de cylindre creux muni d'un filetage pour le vissage osseux, formant une vis dans laquelle un conduit et des gorges sont ménagés pour offrir un espace de greffe permettant l'insertion de tissu ou substitut osseux ou de ciment pour faciliter la fusion des structures dans laquelle la vis est implantée. On notera que l'on désigne ici les apophyses articulaires (ou processus articulaires ou facettes pédiculaires) vertébrales par le terme « facette articulaire », car chaque vertèbre s'articule avec celle du dessus et du dessous par des facettes articulaires qui sont postérieures et l'invention est utile pour le traitement de ces facettes articulaires, mais il est possible d'utiliser éventuellement divers modes de réalisation sur d'autres structures, notamment vertébrales, comme par exemple les facettes costales ou sacro-iliaques si besoin. Les apophyses articulaires font saillie au-dessus et au-dessous de la base des apophyses transverses des vertèbres, en arrière des pédicules. Au niveau lombaire par exemple, les apophyses articulaires supérieures sont séparées l'une de l'autre par une distance plus considérable que celle qui sépare les deux inférieures. Les facettes articulaires qu'elles supportent ont la forme d'une gouttière verticale dont la concavité regarde en arrière et en dedans, gouttière dans laquelle viennent se placer les apophyses articulaires inférieures, qui ont une surface articulaire convexe en sens inverse, c'est-à-dire en avant et en dehors. Les apophyses articulaires inférieures offrent une surface articulaire convexe, en forme de segment de cylindre, qui regarde en dehors et légèrement en avant. Cette surface glisse dans la concavité de l'apophyse articulaire supérieure de la vertèbre située au-dessous. Ces structures sont donc importantes pour la stabilité des vertèbres les unes sur les autres et on notera d'ailleurs que le déficit osseux (ou « lyse ») des isthmes (ou « pars interarticularis ») situés à leur base est souvent responsable de spondylolisthesis (glissement d'une vertèbre par rapport aux autres adjacentes) qui conduisent généralement à une dégénérescence des disques intervertébraux. Lorsque l'on cherche à réaliser une arthrodèse vertébrale, il est donc parfois souhaitable d'utiliser un implant facettaire pour fixer les apophyses articulaires inférieures d'une vertèbre aux apophyses articulaires supérieures de la vertèbre adjacente. Ces implants facettaires peuvent être soit « inter-facettaires », c'est-à-dire qu'ils sont insérés entre les surfaces articulaires, soit « transfacettaires », c'est-à-dire qu'ils sont insérés au travers des apophyses articulaires pour fixer les surfaces articulaires entre elles. Les implants interfacettaires sont généralement mis en place dans le joint articulaire, en identifiant l'axe d'approche et par exemple en positionnant une broche servant de guidage pour l'implant, qui est souvent canulé (i.e., creux). Un problème dans le domaine concerne la solidité puisqu'il est souhaitable de garantir l'intégrité de l'implant malgré sa petite taille et son agencement souvent creux. En général, dans le cas d'une implantation au niveau des facettes articulaires, on préfère utiliser implants de façon à sécuriser les facettes (gauche et droite) qui articulent deux vertèbres adjacentes, mais cette utilisation n'est bien entendu pas limitative.

De manière générale, l'implant facettaire (IF) comporte, d'une part, un corps (80) allongé entre une extrémité libre et une tête (78), selon un axe longitudinal et, d'autre part, des spires (72) d'au moins un filetage, sur au moins une portion dudit corps (80) à proximité de l'extrémité libre, le long de l'axe longitudinal. De plus, le corps (80) de l'implant facettaire (1) comporte de préférence au moins un conduit interne longitudinal (71) sur au moins une portion du corps (80) le long de l'axe longitudinal et/ou des fenêtres (75) traversant les parois du corps depuis l'extérieur du corps jusqu'au conduit interne longitudinal (71) dans un plan, dit transverse, non parallèle à l'axe longitudinal. Ledit corps (80) pouvant être de la forme d'une vis ou d'une cale.

Dans certains modes de réalisation, ce conduit interne longitudinal (71) est obtenu par au moins un premier usinage central parallèle à l'axe longitudinal et au moins un second usinage dans un plan, dit transverse, non parallèle à l'axe longitudinal, et traversant les parois du corps (80) jusqu'au conduit interne longitudinal (71) en ménageant des fenêtres (75) communiquant entre ledit conduit interne longitudinal (71) et l'extérieur du corps (80). Ainsi, l'implant comporte un conduit interne (71) qui préserve au moins une partie desdites spires (72) et la paroi du corps (80) derrière les spires, et en préserve des portions non usinées sur le pourtour dudit corps (80).

Dans certains de ces modes réalisation, le second usinage peut par exemple être réalisé tangentiellement au pourtour du corps (80), résultant en des fenêtres (75) qui s'évasent de l'intérieur vers l'extérieur du corps (80), comme par exemple représenté sur les figures 4A, 4B, 11A et 11B. Cependant, en alternative, le second usinage peut être réalisé sensiblement radialement (ou selon un axe oblique entre l'orientation radiale et l'orientation tangentielle), de façon à obtenir des fenêtres possédant au moins un bord latéral extérieur affuté.

Dans certains modes de réalisation, ladite extrémité libre du corps (80) est auto-foreuse. Par le terme « auto-foreuse », on désigne ici le fait que cette extrémité est capable de forer elle-même du tissu osseux. Une telle définition fonctionnelle peut trouver application simplement par une forme pointue de l'extrémité mais peut également être avantageusement obtenu par une tête fendue ou par le fait qu'une fenêtre (75) est présente sur une portion distale extrême et offre une surface coupante permettant de forer dans du tissu osseux. Dans une autre mode de réalisation, il est possible de prévoir une encoche sur une extrémité cylindrique ou conique ou tronconique pour permettre à l'extrémité libre du corps (80) d'être auto-foreuse. Mais il est également possible de prévoir que la fonction de forage soit obtenue par au moins une fenêtre (75) au niveau de l'extrémité distale. Ainsi, par exemple, on peut ménager une fenêtre (75) qui s'étend sur plusieurs spires (72) et qui offre une tranche coupante permettant d'aviver l'os plus facilement.

Ainsi, dans certains modes de réalisation, lesdites fenêtres (75) sont décalées les unes par rapport aux autres le long (ou plutôt autour) de l'axe longitudinal (non représenté sur les figures), tandis que dans d'autres modes de réalisation, lesdites fenêtres (75) sont alignées entre elles le long de l'axe longitudinal, par exemple représenté sur les figures 4A et 11A. On notera qu'il est également possible de prévoir une combinaison de ces agencements, en prévoyant des fenêtres alignées sur une portion et des fenêtres décalées sur une autre portion. Lorsqu'elles sont décalées entre elles, on préfère généralement qu'une fenêtre plus proximale soit décalée par rapport à une fenêtre plus distale du côté qui correspond au sens du vissage. Ainsi, par exemple, avec un filetage orienté dans le sens horaire, une fenêtre proximale sera décalée à gauche par rapport à une fenêtre plus distale, de sorte à améliorer l'avivement de l'os ou cartilage qui pourra être obtenu progressivement par les fenêtres successives lors du vissage.

Dans certains modes de réalisation, non exclusifs mais cependant indépendants de ceux à deux usinages non parallèles définis ci-dessus, lesdites fenêtres (75) de l'implant interfacettaire (IF) présentent avantageusement au moins un bord extérieur affuté. En effet, quelle que soit la manière dont le conduit et les fenêtres sont obtenus il peut être utile de prévoir au moins un bord extérieur affuté pour les fenêtres (75). En particulier, on préfère généralement que le bord affuté soit celui qui attaque en premier l'os lors du vissage de l'implant, de sorte que ce bord affuté puisse progressivement creuser de l'os (par exemple en découpant des copeaux) pendant le vissage. Ainsi, lorsque les fenêtres (75) sont obtenues par un second usinage, celui peut par exemple être réalisé selon un axe radial ou oblique comme expliqué ci-dessus, de sorte à obtenir au moins un bord d'attaque affuté, comme par exemple représenté sur les figures 4D et 4E.

Ainsi, dans certains modes de réalisation, ladite tête (78) de l'implant (80) ferme le conduit interne longitudinal (71) ou comporte des moyens de fermeture du conduit interne longitudinal (71). De tels moyens de fermeture permettent de prévoir un implant apte à être enfilé sur une broche aidant à l'implantation comme dans l'art antérieur et permet néanmoins de boucher l'implant après l'implantation.

En ce qui concerne le pas du filetage, c'est-à-dire l'espacement des spires le long de l'axe longitudinal, la présente demande prévoit également divers types d'agencements non limitatifs qui peuvent être utiles selon les conditions. En particulier, dans certains modes de réalisation, les spires (72) du filet (ou par extension le filet (72) de l'implant) possède un pas variable se raccourcissant en direction de la tête (78). De même, dans certains modes de réalisation, le corps (80) est muni de plusieurs filets (72) de pas différents, le pas d'un filet situé du côté de l'extrémité libre étant de taille plus importante que le filet adjacent situé du côté de la tête (78).De préférence, le pas d'un filet situé du côté de l'extrémité libre est de taille plus importante que le filet adjacent situé du côté de la tête (78), de sorte que le pas du filet se réduit au fur et à mesure que l'on avance vers la tête. Ce type d'agencements à pas variable permet d'obtenir un effet compressif. En effet, lorsque l'on visse un tel implant à pas variable ou comprenant plusieurs filets à pas décroissants, on obtient un effet de compression, qui est par exemple particulièrement utile dans le cas d'un vissage dans une structure osseuse où l'on souhaite bien plaquer les structures entre elles, comme par exemple une implantation transfacettaire.

D'autre part, dans certains modes de réalisation, non exclusifs mais cependant indépendants de ceux à deux usinages et/ou à bord affuté définis ci-dessus, ladite tête (78) de l'implant (IF) est munie de moyens de stabilisation (e.g., de compression, de verrouillage, d'appui) de l'implant, destinés à prendre appui sur le tissu osseux autour de ladite tête (78) (ces moyens de stabilisation comprenant optionnellement des moyens de verrouillage pour les sécuriser sur l'implant). Divers modes de réalisation sont décrits ci-après pour les moyens de stabilisation mais l'homme de métier comprendra de cette définition fonctionnelle que l'implant est prévu pour que sa tête (qui est généralement la partie qui subsiste à l'extérieur du tissu osseux ou de l'espace articulaire) soit stabilisée sur le tissu osseux (sur une surface osseuse ou sur les bords de l'articulation).Dans certains de ces modes de réalisation, les moyens de stabilisation comportent au moins un élément de stabilisation (8) formant une sorte d'agrafe comprenant au moins deux tiges (81) sensiblement parallèles à l'axe longitudinal et aptes à pénétrer le tissu autour de la tête (78) et éventuellement d'une portion dudit corps (80) à proximité de ladite tête (78). Des exemples de tels moyens de stabilisation (8), tels que représenté sur les figures 12A et 12D, comprenant une bague destinée à être enfilé sur une portion de la tête et au moins une pointe destiné à être plantée dans des tissus environnants. Dans certains de ces modes de réalisation, lesdites tiges (81) de l'élément de stabilisation (8) ont une extrémité libre pointue. Le terme « pointu » dans la présente description désigne en fait plus largement le fait qu'une structure est apte à pénétrer du tissu osseux, en couvrant donc des structures biseautées aussi bien des structures en pointe et de manière non limitative. De plus, dans certains modes de réalisation, lesdites tiges (81) sont reliées entre elles par une bague, telle que décrite dans l'art antérieur WO2016016474, rendant l'élément de stabilisation (8) apte à être monté sur ladite tête (78), mais l'homme de métier appréciera que ce type de montage sur la tête n'est qu'un exemple illustratif et non limitatif puisque divers agencements permettront de fournir des moyens de montage pour les moyens de stabilisation sur la tête (ou toute autre partie de l'implant éventuellement). Selon divers variantes, la bague (83) est enfilée ou vissée sur une portion haute (83) qui dépasse de la tête (78), alors que dans d'autres variantes, la bague repose sur la tête et son ouverture est apte à recevoir une portion basse (83) de moyens de verrouillage (73) qui se fixent dans la tête de l'implant. On notera que les figures montrent des filetages et taraudages pour la fixation des moyens de verrouillage sur la tête, mais que divers types d'agencements sont possibles, comme détaillé ci-dessus en référence aux moyens de fermeture de l'implant.

Dans certains modes de réalisation, en particulier ceux comprenant un élément de stabilisation (8) muni de deux tiges (81) destinés à pénétrer le tissu osseux autour de ladite tête (78), cette dernière comporte au moins deux encoches (88) aptes à recevoir lesdites tiges (81) ou des épaulements (82) disposés le long desdites tiges (81). De telles encoches (88) permettent d'imposer la position des tiges (81) autour de l'implant, de sorte qu'il soit possible de prévoir qu'elles soient idéalement positionnées par rapport aux structures osseuses traitées (notamment pour qu'elles soient plantées chacune dans l'une des vertèbres adjacentes lors d'une implantation inter-facettaire). De plus, la présence d'un épaulement (82) (et de matière entre le centre de l'élément de stabilisation et lesdites tiges) permet de maintenir lesdites tiges à distance du corps (80), de sorte que ces tiges puissent être plantées à une distance assurant une meilleure stabilisation que si elles avaient été plus proches du corps de l'implant.

Dans certains modes de réalisation, lesdits moyens de stabilisation comportent au moins un élément de stabilisation (8) en forme de cloche montée sur (ou solidaire de) la tête (78) et dont le pourtour (81) est destiné à prendre appui sur le tissu osseux entourant la tête (78), comme par exemple représenté sur les figures 5A, 5B et 5C. Dans divers modes de réalisation, la cloche montée solidaire de la tête est formée d'un seul tenant avec la tête ou est fixée dessus. Dans d'autres modes de réalisation, la cloche est montée de manière mobile autour de la tête. De plus, la cloche peut être dans le même matériau que le corps de l'implant (généralement un matériau solide métallique, comme par exemple le titane), mais il est possible de prévoir une cloche dans un autre matériau, notamment plus souple de sorte qu'elle soit écrasée lors du vissage final des moyens de verrouillage et assure ainsi une compression efficace. Un matériau possible et utile pour ce type de variantes de réalisation est le PEEK bien connu dans le domaine.

Dans certains modes de réalisation, la cloche est mobile et permet un appui de type « poly-axial » c'est-à-dire qu'elle peut être verrouillée dans diverses positions par rapport à l'axe longitudinal de l'implant. Par exemple, dans certains de ces modes de réalisation, ladite tête (78) possède une surface inférieure (780) périphérique en forme de portion de sphère, comme par exemple représenté sur les figures 4B, 4D et 4E. Une telle surface est généralement prévue pour être complémentaire d'une surface supérieure interne de ladite cloche (8) ainsi articulée sur la tête (78) de l'implant, comme par exemple représenté sur la figure 4B, de sorte à permettre un ajustement de l'orientation de la cloche par rapport à l'axe de la tête.

Dans certains modes de réalisation, ladite cloche (2) comporte au moins une pointe ou dent sur son pourtour (81) pour faciliter l'ancrage osseux, comme par exemple représenté sur les figures 4A et 4B. Ce type d'agencement de la partie basse de la cloche, destinée à être en appui sur le tissu osseux, permet d'améliorer l'accroche de la cloche sur ce dernier et améliore ainsi la stabilité de l'implant. Dans les modes de réalisation comprenant un conduit interne longitudinal (71) et des fenêtres (75) obtenus par, respectivement, au moins un premier usinage et au moins un second usinage, le second usinage préserve la matière du corps (80) derrière les spires (72), comme par exemple représenté sur les figures 4A, 4B, 11A et 11B. Ainsi, l'homme de métier comprend que l'implant qui en résulte se trouve amélioré par le fait qu'il possède des spires pénétrant plus profondément dans le tissu, comme si elles étaient plus grandes, puisque la matière autour des spires a été enlevées par l'usinage 15 (réduisant ainsi la largeur résiduelle du corps) et que la pression présente dans les tissus environnants, en particulier dans le cas d'une implantation au niveau articulaire, fera que la spire s'enfonce plus profondément dans l'os. De plus, la solidité de l'implant se trouve amélioré grâce à la matière préservée par l'usinage, alors que dans l'art antérieur, seul le filet est 20 préservé et les spires (12) sont seules à supporter les efforts importants pendant et après le vissage. Ainsi, on obtient un implant stable et solide.

Dans ces modes de réalisation, lesdits moyens de stabilisation comportent de préférence des moyens de verrouillage (73) appuyant sur l'élément de stabilisation (8) pour le maintenir appuyé contre le tissu osseux.

D'ailleurs, on notera que dans de nombreux modes de réalisation représentés sur les figures, les fenêtres (75) sont ménagées entre les spires (72) du filetage et généralement entre la totalité (ou la quasi-totalité) des spires. Cependant, il est possible de ménager ces fenêtres que sur une partie des spires. Ainsi, au moins une partie desdites fenêtres (75) sont, par exemple, séparées par au moins deux spires (72) dépourvues de fenêtres (75). Inversement (mais non exclusivement et de manière combinable aux modes détaillés ci-dessus), comme pour l'extrémité libre, il est possible de prévoir sur diverses portions (proximale, médiane ou distale) des fenêtres qui s'étendent sur plusieurs spires plutôt que d'être confinées à l'espace entre deux spires. Ainsi, dans certains modes de réalisation, au moins une partie desdites fenêtres (75) sont ménagées sur plusieurs spires (72).

Les termes « tête » et « extrémité libre » sont utilisés dans la présente description en référence au fait que l'implant se présente généralement sous la forme d'une vis, avec un corps (80) généralement cylindrique ou conique ou tronconique, mais ces termes et ces formes de l'implant ne doivent pas être considérés comme limitatifs. En effet, le pourtour du filetage est sensiblement cylindrique malgré la forme conique ou tronconique du corps de l'implant facettaire (IF). D'autre part, les diverses portions de l'implant sont désignées dans la présente demande par les termes « proximal » signifiant « à proximité de la tête », ou « distal » signifiant « à proximité de l'extrémité libre » ou encore « médiane » signifiant « sensiblement au milieu entre les deux extrémités », mais il est clair que ces termes ne sont pas limitatifs non plus et que l'homme de métier appréciera que la position de ces portions peut varier le long de l'axe longitudinal. De plus, le terme « sensiblement » ou « substantiellement » est utilisé en référence à diverses caractéristiques pour indiquer qu'elles peuvent être exactement comme défini ou être approximativement comme défini. Par exemple, l'expression « une forme sensiblement plane » doit être comprise comme désignant une forme approximativement plane puisque l'homme de métier pourra faire varier la forme exacte dans la mesure où elle conserverait une forme globalement plane répondant aux exigences techniques concernées. De même, la présente description peut définir des caractéristiques sans cette précision d'approximation par les termes « sensiblement » ou « substantiellement », mais il sera clair pour l'homme de métier que cette notion s'applique même en l'absence de tels termes. De plus, le terme « usinage » est utilisé ici de façon non limitative pour désigner la fabrication de l'implant et il est clair que ce terme couvre en fait tout type de techniques de fabrication, tels que, par exemple, les alésages, perçages ou fraisages, mais également l'électroérosion ou tout type de technique permettant de ménager des surfaces ou logements sur ou dans l'implant. De même, le terme « transverse » est utilisé pour indiquer que le second usinage est dans un plan non parallèle à l'axe longitudinal et tend à indiquer qu'il est perpendiculaire à l'axe longitudinal, mais l'homme de métier comprendra, notamment à cause de l'orientation oblique des spires d'un filetage, que ce plan (qui est donc sensiblement transverse) n'est pas forcément perpendiculaire à l'axe longitudinal et sera généralement plutôt orienté obliquement, de préférence parallèlement aux spires.

### Les implants de type interépineux (IE) :

Divers modes de réalisation de l'implant interépineux (IE) selon la présente invention peuvent être utilisés pour l'arthrodèse d'au moins deux vertèbres (VI, VS) adjacentes, comme représenté non-limitativement dans les figures 3A à 3E.

Les implants inter-épineux doivent permettre de redonner de la hauteur entre les épineuses et maintenir cette hauteur (soulager les facettes articulaires, les racines nerveuses, le disque, etc.), notamment dans l'attente d'une arthrodèse (fusion osseuse). Ils doivent être stables entre les épineuses, notamment au niveau des lombaires, en disposant d'un moyen d'accroche ou non sur les épineuses. De plus, on souhaite généralement disposer de différentes hauteurs et/ou largeurs et/ou profondeurs des implants, notamment pour les adapter au mieux à leur site d'implantation. De plus, on préfère, dans certains cas, immobiliser les deux épines, alors que dans d'autres cas, on préfère conserver une mobilité. On souhaite également parfois, contrôler l'étendue de la mobilité, notamment les mouvements de flexion et de rotation des vertèbres.

La présente invention concerne un implant inter-épineux (IE) destiné à être implanté entre les apophyses épineuses de deux vertèbres adjacentes. On désigne dans la présente demande les apophyses épineuses ou processus épineux des vertèbres par le terme « épine vertébrale », « épine dorsale », « épineuse » ou tout simplement « épine ». Les épines sont les structures les plus postérieures du rachis et donc potentiellement les plus rapides d'accès (on peut d'ailleurs les palper sous la peau de C7 à L5), ce qui rend certains implants inter-épineux faciles à implanter mais peut imposer des contraintes de stabilité et d'implantation exposées dans la présente demande. On désigne ainsi dans la présente demande l'espace qui sépare deux épines adjacentes par le terme d'espace inter-épineux. Certains modes de réalisation permettent l'insertion de plusieurs implants entre les espaces inter-épineux successifs de plusieurs (plus que deux) vertèbres adjacentes, comme détaillé dans la présente demande.

Les épines dorsales (El, ES), généralement sensiblement alignées dans le plan sagittal médian (ou approximativement orientée dans ce plan), présentent sensiblement une forme de plot ou de plaque, de section coronale généralement oblongue ou elliptique, avec une extrémité (une crête) pointant vers l'arrière du patient. Les épines possèdent, en référence à l'axe longitudinal du rachis, un bord supérieur (E2), un bord inférieur (E3) et deux faces latérales opposées (E4, E5).

La figure 8E représente de façon schématique des vues en perspective des éléments constitutifs de deux vertèbres adjacentes (VI, VS), avec le plan sagittal médian (A) et un repère cartésien (X, Y, Z) qui sont utilisés comme références dans la présente description par souci de simplification. Ces figures représentent une vertèbre inférieure (VI) avec son épine, dite également inférieure (EI), et une vertèbre supérieure (VS) avec son épine, dite également supérieure (ES) et montrent les bords (E2, E3) et faces (E4, E5) de chacune de ces épines supérieure (ES) et inférieure (El). La présente demande décrit certains éléments de l'implant en référence à son positionnement une fois implanté dans le corps du patient, pour plus de clarté sur la localisation des divers éléments de l'implant par rapport aux structures vertébrales. Dans la figure 8E, le patient est considéré en position horizontale, allongé sur le ventre. Le repère cartésien représenté comporte trois directions (X, Y, Z) orthogonales entre elles. La direction X correspond à l'axe longitudinal ascendant du rachis, la direction Y correspond à l'axe antéropostérieur et la direction Z correspond à l'axe médio-latéral. Ainsi, les axes X et Y définissent un plan sagittal (A), les axes X et Z définissent un plan coronal et les axes Y et Z définissent un plan transversal du patient. La présente demande pourra référer à ces plans du patient pour l'implant lui-même. Les termes « supérieur » et « inférieur » sont donc utilisés dans la présente demande en référence à l'axe X. Une vue de dessus est une vue selon laquelle on regarde le rachis ou l'implant depuis l'arrière du patient allongé sur le ventre et une vue latérale est une vue selon laquelle on regarde le rachis ou l'implant depuis une face latérale du rachis ou de l'implant selon cette même référence. Ces considérations de vues et d'orientations sont utilisées indépendamment que l'implant soit implanté ou non et l'on comprendra qu'elles concernent l'implant dans sa destination d'implantation mais qu'elles ne sont pas limitatives. De plus, on comprendra également de la présente demande que l'implant peut être retourné selon la face latérale du rachis par laquelle le chirurgien souhaite aborder l'espace inter-épineux. D'autre part, on comprendra que l'invention permet d'aborder les épines par une seule face latérale du rachis mais que l'abord ne se fait de préférence pas perpendiculairement au plan sagittal (selon l'axe Z) mais plutôt sensiblement parallèlement à l'axe Y ou de préférence selon un axe oblique qui est orienté vers l'arrière du patient (un axe orienté entre les axes Y et Z, mais également éventuellement non parallèle à l'axe X si nécessaire). En revanche, on comprendra que les insertions de l'implant et de l'insert se font de préférence sensiblement parallèlement à l'axe Z. Enfin, toujours en référence à ces repères sur le patient, on utilise dans la présente demande les termes « hauteur » (notamment de l'implant) ou « longueur » (notamment des ailes ou de l'insert) pour désigner une dimension selon la direction X, les termes « largeur » (notamment de l'implant) ou « épaisseur » (notamment des ailes ou de l'insert) pour désigner une dimension selon la direction Z et le terme « profondeur » pour désigner une dimension selon l'axe Y. Ces termes utilisés pour les dimensions selon les axes et directions du repère ne sont pas limitatifs et ne signifient pas que les éléments en question soient forcément orientés exactement selon l'axe auquel il est fait référence et cette référence pourra concerner leur projection orthogonale sur l'axe ou leur dimension selon un axe approximativement orienté dans la même direction que l'axe auquel il est fait référence. La présente invention concerne un implant interépineux (IE), destiné à être implanté entre deux épines vertébrales (El, ES) desdites vertèbres (VI, VS), l'implant interépineux comportant au moins deux ailes (21, 22, 31, 34) de dimensions agencées pour être inséré entre deux épines (El, ES) vertébrales desdites vertèbres (VI, VS) adjacentes, depuis une de leurs faces latérales ;
Le terme « aile » désigne dans la présente demande un élément de l'implant (IE) qui s'étend en direction des faces latérales des épines (en général longitudinalement et sensiblement parallèle à l'axe X du rachis) et ne doit pas être interprété de façon limitative car les ailes peuvent avoir diverses formes, dont divers exemples sont détaillés dans la présente demande. On notera en particulier que les ailes sont en fait des extensions du corps (par exemple sur les faces latérales ou sur les faces supérieure et inférieure, ou plutôt à la jonction entre ces faces). Certaines ailes sont articulées et comportant dans un mécanisme de verrouillage pour l'articulation de façon à remplir leur fonction de retenue de l'implant et permettant généralement de fournir avantageusement un moyen de compression. D'une manière générale les ailes (que l'on pourrait appeler bras, jambe, extension ou autrement) pourront être en forme de plaque droite ou courbe et auront de préférence des formes et dimensions adaptées à un bon maintien de l'implant entre les épines. Par exemple dans le cas d'ailes courbes, l'insertion de l'implant sera facilité et l'extrémité libre des ailes courbes se trouvera plus proche des faces latérales des épines que le reste de l'implant, voire même au contact de ces faces latérales pour permettre un bon maintien de l'implant entre les épines. Les dimensions des ailes pourront également être adaptées aux dimensions des faces latérales des épines. Par exemple, les ailes pourront avoir une longueur (sensiblement parallèle à l'axe X du rachis), de l'ordre de la moitié de celle des faces latérales des épines, voire supérieure (ce qui sera particulièrement avantageux pour fournir un bon maintien, et sera possible même dans certains modes de réalisation où les ailes ont des formes agencées pour permettre une implantation de plusieurs implants dans les espaces inter-épineux successifs de plusieurs vertèbres successives du rachis, par exemple comme détaillé dans la présente demande). La profondeur de l'implant d'une manière générale pourra varier en fonction de la taille des épines selon un axe sensiblement parallèle à l'axe Y du rachis et/ou du souhait du chirurgien, pour offrir un maintien plus ou moins important des épines, grâce à un contact avec une surface plus ou moins étendu sur les bords supérieur et inférieur des épines. Cette profondeur pourra également être déterminée en fonction de l'encombrement total souhaité de l'implant, pour minimiser les lésions nécessaires à son implantation. Ainsi, l'implant pourra avoir une profondeur ainsi déterminée et les ailes (31, 32) pourront avoir une profondeur sensiblement identique ou différente, selon les besoins définis par le chirurgien. De plus, le terme « aile » est utilisé ici pour désigner une structure faisant saillie depuis un bord ou un angle du corps de l'implant et qui s'étend de façon à ce que les dimensions de l'implant empêche que les ailes puissent passer librement entre les deux épineuses par un simple translation selon un axe parallèle à l'axe Z, du moins lorsque les ailes sont déployables. Mais comme décrit plus haut, dans le cas d'une résection partielle des épineuses, il est aussi envisageable d'avoir des ailes fixe et une implantation de l'implant interépineux par translation selon un axe parallèle à l'axe Y. Le terme « aile » ne doit pas être considéré comme limitatif. De plus, il est fait référence, dans la présente demande, à au moins deux ailes déployables et il est donc possible de prévoir quatre ailes déployables ou deux ailes fixes et deux ailes déployables, mais aussi seulement deux ailes fixes, notamment lorsque l'implant interépineux est muni de moyens d'immobilisation, par exemple comme décrit ci-après. Enfin, les termes « déployer ou déployable ou déploiement » désignent, dans la présente demande, aussi bien un pivotement, comme par exemple illustré de manière non-limitative sur les figures 3A à 3C de la présente demande, qu'une translation d'un insert, comme par exemple illustré de manière non-limitative sur les figures 1, 4 et 6 de la demande de l'art antérieur US2005203512, ou une combinaison des deux.

Dans certains modes de réalisation, lesdits implants interépineux (IE) comprennent des moyens d'immobilisation de sorte à immobiliser la structure vertébrale dans laquelle il est configuré pour être implanter.

Dans certains modes de réalisation, lesdits moyens d'immobilisation sont des moyens de crochetage d'épineuse agencé pour aller se crocheter autour d'au moins une partie du bord d'une épine qui est opposé au bord de cette épine contre lequel le corps de l'implant est apposé. Ledit moyen de crochetage d'épineuse comporte au moins un crochet monté pivotant sur le corps de l'implant et agencé pour se crocheter autour d'une épine, au niveau d'au moins une partie du bord qui est opposé au bord contre lequel le corps de l'implant est apposé. Ledit moyen de crochetage est décrit dans la demande WO2013001097 et illustré de manière non-limitative sur les figures 20 à 23 de ladite demande. Dans ce type de mode de réalisation, l'implant interépineux, implanté dans l'espace interépineux, est fixé autour des épines et les immobilise, ce qui facilite la fusion.

Dans certains modes de réalisation, lesdits moyens d'immobilisation sont des moyens de fixation. Lesdits moyens de fixation peuvent être des éléments pénétrant dans les épines dorsales, notamment, tels qu'une vis ou un clou ou au moins une plaque. Dans le cas de clou ou de plaque, on préfère qu'ils soient courbés pour limiter les risques de mouvement. Lesdits moyens de fixation peuvent également être des éléments entourant les épines dorsales de façon à immobiliser lesdites épines, tel qu'un ligament par exemple. Ce type de mode de réalisation permet donc également d'immobiliser l'implant interépineux dans l'espace interépineux et faciliter la fusion osseuse.

Dans certains modes de réalisation, lesdits moyens d'immobilisation sont des moyens de compression des faces latérales d'au moins une épineuse. De préférence, il est prévu une compression des épineuses par un pivotement d'au moins une aile de l'implant ou d'un insert de l'implant pour presser l'épineuse entre cette aile pivotante et au moins un autre aile, fixe ou également pivotante. Ce type de mode de réalisation comprenant un moyen de compression des épineuses (par exemple et en particulier en appliquant une pression sur les faces latérales) permet de stabiliser l'implant interépineux dans l'espace interépineux et faciliter la fusion osseuse,

Dans certains modes de réalisation, le système comporte au moins un implant interépineux (IE), comportant au moins un corps (2) de dimensions agencées pour être inséré entre deux épines (El, ES) vertébrales desdites vertèbres (VI, VS) adjacentes, depuis une de leurs faces latérales, et comportant, d'une part, au moins deux ailes (21, 22) s'étendant non parallèlement au corps (2) de sorte qu'au moins une portion de chaque aile (21, 22) longe au moins une portion latérale d'une des deux épines (El, ES) et, d'autre part, au moins un passage (28) traversant ledit corps (2) d'une face latérale à l'autre et recevant au moins un moyen de retenue de l'implant interépineux (IE), formé par un insert (3), inséré depuis la même face latérale de l'implant et comprenant au moins une aile courbe et rigide (31, 32) coulissant dans ledit passage (28) sans déformation jusqu'à ce qu'au moins une portion desdites ailes (3) courbe et rigide (31, 32) projette à l'extérieur dudit passage (28) et longe au moins une portion d'une face latérale opposée à celle longée par une aile (21, 22) de l'implant. L'insert comprend au moins une aile courbe et rigide avec une forme de section rectangulaire, circulaire, carrée, polygonale ou en T, en L, en U ou même en H. Divers modes de réalisation de l'implant inter-épineux présentent un corps longitudinal (2) comportant au moins deux ailes (21, 22) disposées sur une même face latérale de l'implant (IE) pour longer les deux épines (EI ,ES) sur la même face latérale, ledit passage (28) traversant l'implant depuis cette même face latérale jusqu'à la face latérale pour l'insertion d'un insert (3) comprenant deux ailes courbes (31, 32) projetant chacune vers une des épines (El, ES) pour longer la même face latérale opposée, de sorte que l'implantation de l'implant et de l'insert (3) entre les épines (El, ES) puisse être effectuée depuis une seule face latérale.

Selon un mode de réalisation de la présente invention, l'implant(IE) comporte deux ailes (21, 22) disposées chacune sur une face latérale de l'implant opposée à l'autre aile et projetant chacune vers une des deux épines, de sorte que les ailes longent chacune une épine (El, ES) mais sur des faces latérales (E4, E5) opposées, l'insert (3) étant de forme sensiblement sigmoïdale par le fait que son corps comporte au moins deux rayons de courbures d'orientations opposées, de sorte que deux faces dudit corps comportent chacune une portion concave et une portion convexe, le passage (28) et l'insert (3) étant agencés de sorte que, lorsque l'insert (3) est logé dans le passage (28), au moins une partie desdites portions convexes des deux faces de l'insert (3) longent chacune au moins une portion des épines (El, ES), sur des faces latérales opposées à celles longées par les ailes (21, 22).

Dans un autre mode de réalisation, l'implant interépineux (IE) comporte des moyens de compression (31, 32, 35) pour comprimer les faces latérales des épineuses (El, ES) entre lesdites ailes (21, 22) et ledit insert (3) lorsque ce dernier est inséré au travers dudit passage (28) de l'implant interépineux (IE). Lesdits moyens de compression sont formés par deux ailes courbes (31, 32) de l'insert (3) reliées par un axe d'articulation (35) permettant le déploiement des deux corps depuis une position repliée lors de l'insertion de l'insert (3) dans le passage (28) à une position déployée où lesdites ailes (31, 32) compriment les épineuses contre les ailes (21, 22) de l'implant (IE).

Comme représenté dans les figures 3C, 3D et 3E, lesdites ailes (31, 32) de l'insert (3) sont reliées par une articulation fournissant un moyen de compression à leur extrémité postérieure et permettant le déploiement des deux ailes (31, 32) d'une position repliée où les ailes sont au contact ou proche l'une de l'autre, telle que représentée sur les figures 3A et 3D, à une position déployée où les ailes sont écartées l'une de l'autre telle que représentée sur les figures 3C et 3E. Le déploiement des deux ailes (31, 32) a lieu, dans ces modes de réalisation, grâce à cette articulation et au contact des ailes au fond du passage (28) (i.e., au moins une paroi située dans l'axe du passage et opposée à l'entrée de ce dernier) et le degré de déploiement (i.e., l'orientation des inserts en position déployée) dépendent donc de la position de l'articulation dans le passage. Un tel moyen de compression de l'articulation forme de préférence un axe d'articulation (35) sur lequel sont montées, libres en rotation, les extrémités postérieures des ailes (31, 32) de l'insert (3), par exemple grâce à des oeillets (34) formés à ces extrémités postérieures. L'axe d'articulation (250), par exemple formé par une tige, une vis, un rivet ou une autre structure peut, à une première extrémité, former une butée pour retenir l'une des ailes (31 ou 32) en translation le long de l'axe (250), l'autre aile (31 ou 32) étant alors également retenu par son imbrication avec le premier.

Dans certains modes de réalisation, l'implant interépineux (IE) comporte d'une part, au moins deux ailes (31, 32) s'étendant de sorte qu'au moins une portion de chaque aile (31, 32) longe au moins une portion d'une face latérale d'une des deux épines (El, ES) et d'autre part, au moins un passage (28) traversant au moins une portion du corps (2). Dans divers modes de réalisation, ce passage possède une forme, des dimensions et une orientation agencées pour l'insertion, au travers du corps (2), d'au moins un insert (3). Dans divers modes de réalisation, l'insert (3) comprend au moins une aile courbe retenue dans le corps (2) de sorte qu'au moins une portion de ladite aile courbe longe au moins une portion d'une face latérale opposée à (au moins) une face latérale longée par une aile (31, 32) (une face latérale ou deux faces latérales comme détaillé dans ledit mode de réalisation). On comprend que l'on obtient ainsi au moins un implant muni d'au moins une aile longeant une face latérale d'une épine dont l'autre face latérale est longée par une portion d'au moins un insert formant au moins une autre aile déployée au travers d'un passage de l'implant. Dans ces modes de réalisation, cette configuration permet donc d'insérer l'implant depuis une face latérale en vis-à-vis de laquelle sera disposée une aile et ensuite d'insérer dans le passage (28) de l'implant, par la même face latérale, un insert (3) dont une portion courbe permet de venir au moins en vis-à-vis de la face latérale opposée (située de l'autre coté de l'épine et à laquelle on n'a donc pas d'accès direct) et potentiellement une autre portion courbe du côté depuis lequel l'insert est inséré.

Suivant un mode de réalisation, l'insert (3) est retenu dans l'implant, voire même y est fixé. Des moyens de butée sont décrits dans la présente demande comme exemples de mécanisme de butée (ou de fixation) de l'insert. Cependant, dans certains modes de réalisation, l'insert (3) est retenu dans l'implant sans moyens de butée. Par exemple, dans certains modes de réalisation, l'insert (3) peut être retenu dans l'implant grâce à une portion épaissie de l'insert qui vient au contact des parois dans le passage de l'implant, de sorte que l'insert est rentré en force dans le passage et s'y trouve maintenu par frottement. De même, plutôt qu'un épaississement, il est possible que l'insert soit retenu dans l'implant par le fait que sa courbure soit tellement importante par rapport aux dimensions du passage qu'il doive être rentré en force dans ce dernier et s'y trouve maintenu par son contact avec les parois du passage. On préférera alors un insert peu flexible pour éviter qu'il ne puisse trop facilement se déloger du passage en se déformant sous l'effet de contraintes. Néanmoins, pour assurer une bonne retenue de l'insert, divers mécanismes de butée comprenant des moyens de butée spécifiques sont également envisagés.

Dans certains modes de réalisation, en particulier ceux où l'implant (IE) comporte deux ailes sur la même face latérale, le corps comporte de préférence, mais à titre non limitatif, sur sa partie antérieure (destinée à être insérée en premier), au moins un chanfrein (24) pour faciliter son insertion dans l'espace inter-épineux (en particulier au travers du ligament inter-épineux). Par exemple, comme particulièrement visible sur les figures 3A, et 3B, le corps pourra être muni de chanfreins (24) à l'extrémité antérieure des faces supérieures et inférieure, mais également des faces disposées en direction de l'avant et de l'arrière du patient.

Dans certains modes de réalisation non représentés, des moyens de crochetage d'épineuse (El, ES), non représenté sur les figures, sont prévus pour améliorer la retenue de l'implant entre les deux épines, en crochetant au moins une des deux épines adjacentes entre lesquelles l'implant est disposé et ils fournissent alors une fonction de retenue des épineuses en plus de la fonction de retenue de l'implant. On entend ici par « moyens de crochetage » des moyens qui sont agencées pour aller se crocheter autour d'au moins une partie des bords des épines qui sont opposés aux bords des épines entre lesquelles l'implant est inséré.

La présente demande décrit diverses caractéristiques techniques et avantages en référence aux figures et/ou à divers modes de réalisation. L'homme de métier comprendra que les caractéristiques techniques d'un mode de réalisation donné peuvent en fait être combinées avec des caractéristiques d'un autre mode de réalisation à moins que l'inverse ne soit explicitement mentionné ou qu'il ne soit évident que ces caractéristiques sont incompatibles ou que la combinaison ne fournisse pas une solution à au moins un des problèmes techniques mentionnés dans la présente demande. De plus, les caractéristiques techniques décrites dans un mode de réalisation donné peuvent être isolées des autres caractéristiques de ce mode à moins que l'inverse ne soit explicitement mentionné.

Il doit être évident pour les personnes versées dans l'art que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes, et l'invention ne doit pas être limitée aux détails donnés ci-dessus.

## Revendications

1. Système d'arthrodèse rachidienne comprenant plusieurs implants de plusieurs types, pour l'arthrodèse d'au moins deux vertèbres (VI, VS) adjacentes,
**caractérisé en ce qu'**il comporte, d'une part:
- au moins un implant intersomatique (IS), destiné à être implanté dans l'espace discal entre lesdites vertèbres (VI, VS) adjacentes pour maintenir une distance entre elles et comportant au moins un dispositif d'ancrage osseux (1) pour ancrer ledit implant intersomatique (IS) dans chacune desdites vertèbres (VI, VS) et les immobiliser l'une par rapport à l'autre et, d'autre part, au moins un implant parmi les deux types d'implants suivants :
- au moins un implant de type interépineux (IE), comprenant un corps (2) de dimensions configurées pour maintenir un écartement entre deux épines vertébrales (EI,ES) desdites vertèbres (VI, VS) adjacentes, l'implant interépineux (IE) comportant au moins deux ailes (21, 22, 31, 33) aptes à longer, chacune, au moins une portion d'au moins une face latérale d'au moins une desdites épines (E1, ES) ;
- au moins un implant de type facettaire (IF), allongé selon un axe longitudinal entre une extrémité libre et une tête (78) et destiné à être implanté entre et/ou à travers les facettes articulaires des vertèbres (VI, VS), ledit implant de type facettaire (IF) comprenant au moins une cale munie d'au moins une fixation osseuse ou seulement cette fixation osseuse, ladite cale et/ou ladite fixation osseuse comportant au moins un conduit interne longitudinal (71) dans au moins une portion le long de l'axe longitudinal, et/ou des fenêtres (75) traversant ladite cale et/ou ladite fixation osseuse transversalement à l'axe longitudinal et/ou des moyens de stabilisation au niveau de la tête (78) pour prendre appui sur les tissu osseux environnants.

2. Système selon la revendication 1, **caractérisé en ce que** ladite fixation osseuse de l'implant facettaire (IF) est formé par une ancre rigide, de préférence courbe, ou par une vis munie de spires (72) d'au moins un filetage, sur au moins une portion à proximité de l'extrémité libre.

3. Système selon la revendication 2, **caractérisé en ce que** l'implant intersomatique (IS) est un implant de type postérieur ou transforaminal.

4. Système selon l'une des revendications 1 à 3, **caractérisé en ce que** ledit implant intersomatique (IS) comporte au moins une paroi périphérique, dont au moins une partie, dite postérieure, comporte au moins un passage (40), dimensions et orientation complémentaires aux formes et dimensions dudit dispositif d'ancrage (1) comportant au moins un corps (10) rigide et allongé selon un axe longitudinal s'étendant entre une première extrémité, dite antérieure, et une seconde extrémité, dite postérieure, ledit corps (10) étant inséré sans déformation dans ledit passage (40), sensiblement dans le plan de l'implant intersomatique (IS), par coulissement depuis ladite partie postérieure de l'implant intersomatique (IS), ledit passage (40) traversant l'implant intersomatique (IS) depuis la périphérie vers une surface supérieure ou inférieure de sorte que l'extrémité antérieure du dispositif d'ancrage (1) pénètre dans une desdites vertèbres (VI, VS) adjacentes, tandis que l'extrémité postérieure du dispositif d'ancrage (1) reste dans ledit passage (40) et retient ledit implant intersomatique (IS) contre ladite vertèbre (VI, VS).

5. Système selon la revendication précédente, **caractérisé en ce que** ledit corps (10) du dispositif d'ancrage (1) est courbe de sorte à pouvoir pénétrer une des vertèbres adjacentes (VI, VS) en étant introduit dans l'implant intersomatique (IS) selon un axe d'approche sensiblement dans le même plan que celui de l'implant intersomatique (IS).

6. Système selon l'une quelconque des revendications 4 et 5, **caractérisé en ce qu'**au moins une des surfaces supérieure et inférieure de la paroi périphérique comporte des crans (42) évitant le déplacement de l'implant intersomatique (IS) entre les vertèbres (VI, VS) entre lesquelles il est destiné à être implanté.

7. Système selon l'une quelconque des revendications 4 à 6, **caractérisé en ce que** ledit corps (10) du dispositif d'ancrage(1) comporte au moins une nervure (16) ou seconde plaque coopérant avec au moins une rainure ménagée dans le passage (40) de l'implant.

8. Système selon l'une quelconque des revendications 4 à 7, **caractérisé en ce que** ladite extrémité postérieure de ledit corps (10) du dispositif d'ancrage (1) comporte au moins une butée (13) pressant l'implant intersomatique (IS) contre ladite vertèbre (VI, VS).

9. Système selon l'une quelconque des revendications 4 à 8, **caractérisé en ce que** ledit corps (10) du dispositif d'ancrage (1) comporte au moins une butée (12) s'opposant au retrait dudit dispositif d'ancrage (1) de l'implant intersomatique (IS).

10. Système selon l'une quelconque des revendications 4 à 9, **caractérisé en ce que** ladite extrémité postérieure de ledit corps (10) du dispositif d'ancrage (1) comporte au moins une butée (14) orientée non parallèlement à l'axe longitudinal du corps (10) et complémentaire d'au moins une butée (49) d'au moins un moyen de verrouillage (4) du dispositif (1) par rapport à l'implant intersomatique (IS), ledit moyen de verrouillage (4) qui équipe l'implant (IS) étant muni d'au moins une portion flexible permettant, d'une part, de repousser ladite butée du moyen de verrouillage (4) pour l'insertion du dispositif d'ancrage (1) dans le passage (40), et d'autre part, l'engagement réciproque des deux butées (14, 49) lorsqu'elles se retrouvent l'une en face de l'autre, par le retour élastique de la portion flexible.

11. Système selon l'une quelconque des revendications 4 à 10, **caractérisé en ce que** les plans moyens passant par les surfaces supérieure et inférieure de l'implant intersomatique (IS) forment un angle orienté selon un axe antéropostérieur de l'implant intersomatique (IS) permettant d'imposer une lordose ou une cyphose aux vertèbres entre lesquelles l'implant intersomatique (IS) est destiné à être implanté.

12. Système selon l'une quelconque des revendications 4 à 11, **caractérisé en ce que** la paroi périphérique comporte deux passages (40) orientés chacun vers une des surfaces supérieure et inférieure, de façon à permettre l'ancrage du dispositif d'ancrage (1) dans chacune des vertèbres adjacentes entre lesquelles l'implant intersomatique (IS) est destiné à être implanté.

13. Système selon l'une quelconque des revendications 4 à 12, **caractérisé en ce que** la paroi périphérique comporte au moins un chanfrein (46) sur au moins une portion périphérique d'au moins une de ses surfaces supérieure et inférieure, de façon à faciliter l'insertion de l'implant intersomatique (IS) entre les vertèbres.

14. Système selon l'une des revendications précédentes, **caractérisé en ce que** ledit implant interépineux (IE) comprend des moyens d'immobilisation d'au moins une des épines (El, ES) entre lesquelles il est destiné à être implanté.

15. Système selon la revendication 14, **caractérisé en ce que** lesdits moyens d'immobilisation sont des moyens de fixation ou d'ancrage dans au moins une des épines (El, ES).

16. Système selon l'une des revendications 14 et 15, **caractérisé en ce que** lesdits moyens d'immobilisation sont des moyens de crochetage d'au moins une des épines (El, ES).

17. Système selon l'une des revendications 14 à 16, **caractérisé en ce que** lesdits moyens d'immobilisation sont des moyens de compression d'au moins une des épines (El, ES).

18. Système selon l'une des revendications 1 à 17, **caractérisé en ce que** l'implant interépineux (IE) comporte, d'une part, deux ailes (21, 22) disposées chacune sur une face latérale de l'implant opposée à l'autre aile et projetant chacune vers une des deux épines, de sorte que les ailes longent chacune une épine (El, ES) mais sur des faces latérales (E4, E5) opposées, et d'autre part, au moins un passage (28) traversant ledit corps (2) d'une face latérale à l'autre et recevant au moins un moyen de retenue de l'implant interépineux (IE), formé par un insert (3), inséré depuis la même face latérale de l'implant, l'insert (3) étant de forme sensiblement sigmoïdale par le fait qu'il forme une aile comportant au moins deux rayons de courbures d'orientations opposées, de sorte que deux faces de l'aile comportent chacune une portion concave et une portion convexe, le passage (28) et l'insert (3) étant agencés de sorte que, lorsque l'insert (3) est logé dans le passage (28), au moins une partie desdites portions convexes des deux faces de l'insert (3) longent chacune au moins une portion des épines (El, ES), sur des faces latérales opposées à celles longées par les ailes (21, 22).

19. Système selon la revendication précédente, **caractérisé en ce que** l'implant interépineux (IE) comporte des moyens de compression (31, 32, 35) pour comprimer les faces latérales des épineuses (El, ES) entre lesdites ailes (21, 22) de l'implant interépineux (IE) et ledit insert (3) lorsque ce dernier est inséré au travers dudit passage (28) de l'implant interépineux (IE).

20. Système selon les revendications 18 et 19, **caractérisé en ce que** les moyens de compression sont formés par deux ailes courbes (31, 32) de l'insert (3) reliées par un axe d'articulation (35) permettant le déploiement des deux ailes depuis une position repliée lors de l'insertion de l'insert (3) dans le passage (28) à une position déployée où lesdites ailes (31, 32) compriment les épineuses contre les ailes (21, 22) de l'implant interépineux (IE).

21. Système selon l'une quelconque des revendications précédentes, **caractérisé en ce que** l'insert (3) de l'implant interépineux (IE) est retenu dans le corps (2) par au moins un mécanisme de butée.

22. Système selon l'une des revendications 1 à 21, **caractérisé en ce que** ledit conduit interne (71) de l'implant facettaire (IF) est obtenu par au moins un premier usinage central parallèle à l'axe longitudinal et les dites fenêtres (75) de l'implant facettaire (IF) sont obtenues par au moins un second usinage dans un plan, dit transverse, non parallèle à l'axe longitudinal, de sorte que lesdites fenêtres (75) préservent au moins une partie desdites spires (72) et la paroi du corps derrière les spires, et préservent des portions non usinées sur le pourtour dudit corps de l'implant facettaire (IF).

23. Système selon la revendication 25, **caractérisé en ce que** dites fenêtres (75) de l'implant facettaire (IF) comportent au moins un bord latéral extérieur affuté.

24. Système selon les revendications 25 à 26, **caractérisé en ce que** ladite extrémité libre du corps de l'implant facettaire (IF) est auto-foreuse.

25. Système selon l'une quelconque des revendications précédentes, **caractérisé en ce que** lesdites fenêtres (15) sont alignées entre elles le long de l'axe longitudinal.

26. Système selon la revendication 25, **caractérisé en ce que** le second usinage ménage des fenêtres (15) alignées traversant les parois du corps (10) jusqu'au conduit interne longitudinal (11), de sorte que la taille de l'implant transversalement à l'axe longitudinal soit localement réduite.

27. Système selon l'une quelconque des revendications précédentes, **caractérisé en ce que** lesdits moyens de stabilisation comportent au moins un élément de stabilisation (8) comprenant au moins deux tiges (81) ayant une extrémité libre pointue et sensiblement parallèles à l'axe longitudinal et aptes à pénétrer le tissu autour de la tête (78) et éventuellement d'une portion dudit corps de l'implant facettaire (IF) à proximité de ladite tête (78).

28. Système selon l'une quelconque des revendications 28 à 29, **caractérisé en ce que** lesdites tiges sont reliées entre elles par une bague rendant l'élément de stabilisation apte à être monté sur ladite tête (78).

29. Système selon la revendication 28, **caractérisé en ce que** lesdits moyens de stabilisation comportent au moins un élément de stabilisation (8) en forme de cloche montée sur la tête (78) et dont le pourtour est destiné à prendre appui sur le tissu osseux entourant la tête (78).

30. Système selon la revendication 31, **caractérisé en ce que** ladite cloche (2) comporte au moins une pointe ou dent sur son pourtour (82) pour faciliter l'ancrage osseux.

31. Système selon l'une quelconque des revendications 31 et 32, **caractérisé en ce que** ladite cloche (8) est montée solidaire de la tête (78).

32. Système selon la revendication 30, **caractérisé en ce que** ladite tête (78) possède une surface inférieure périphérique en forme de portion de sphère et complémentaire d'une surface supérieure interne de ladite cloche (8) ainsi articulée sur la tête (78) de l'implant.

33. Système selon l'une quelconque des revendications 30 à 34, **caractérisé en ce que** lesdits moyens de stabilisation comportent des moyens de verrouillage appuyant sur l'élément de stabilisation pour le maintenir appuyé contre le tissu osseux.
